# EUROPEAN PATENT SPECIFICATION

(11) **EP 0 906 274 B1**
(45) Date of publication and mention of the grant of the patent: **13.06.2001**
(21) Application number: 97920414.6
(22) Date of filing: 16.04.1997
(51) Int. Cl.: C07C 303/24, C07C 305/06, C07C 305/10

(54) **PROCESS FOR MANUFACTURING SULPHATES OF LONGER CHAIN BRANCHED ALKANOLS AND ALKOXYLATED ALKANOLS**
VERFAHREN ZUR ERZEUGUNG VON SULFATEN VON LÄNGERKETTIGEN VERZWEIGTEN ALKANOLEN UND ALKOXYLIERTEN VERZWEIGTEN ALKANOLEN
PROCEDE POUR FABRIQUER DES SULFATES D'ALCANOLS ET D'ALCANOLS ALCOXYLES RAMIFIES A CHAINE PLUS LONGUE

(30) Priority: 16.04.1996 US 15521 P; 16.04.1996 US 15523 P; 26.11.1996 US 31761 P
(43) Date of publication of application: 07.04.1999
(73) Proprietor: THE PROCTER & GAMBLE COMPANY, Cincinnati, Ohio 45202 (US)
(72) Inventor: CONNOR, Daniel, Stedman, Cincinnati, OH 45251 (US); CRIPE, Thomas, Anthony, Loveland, OH 45140-7345 (US); JACOBS, Roger, Craig, Hamilton, OH 45013 (US); JENSEN, Michael, Chris, Cincinnati, OH 45209 (US)
(74) Representative: Peet, Jillian Wendy
(86) International application number: US9706338
(87) International publication number: WO9738972

(56) References cited:
- EP-A- 0 401 642
- GB-A- 719 445

## Description

The present invention relates to methods for producing longer chain length alkyl sulfate and/or alkyl alkoxylated sulfate surfactant compositions. This method utilizes the presence of a significant amount of mid-chain branched alcohol and/or mid-chain branched polyoxyalkylene alcohol in the sulfation reaction to significantly reduce the reaction temperature, thereby improving product quality and saving energy.

### BACKGROUND OF THE INVENTION

Sulfonation/sulfation of anionic surfactants worldwide is done on a very large scale, on the order of millions of tons each year. Such processes are well defined and are thoroughly characterized in the art. [See for example, Everett E. Gilbert, "Sulfation and Related Reactions", copyright 1965 by John Willey and Son, Inc., pages 345-354; and "Sulfonation Technology in the Detergent Industry", by W. Herman de Groot, copyright 1991 by Kluwer Academic Publishers, especially pages 213-215.]

The art recognizes that for lower chain length alcohols, such as lauryl alcohol, the feed temperature for the reaction can be kept low, in the 25-30°C range. However, longer chain length alcohols, like the C₁₆₋₁₈ tallow alcohol, require feed temperatures in the 55-60° range. At such higher temperatures, in addition to the added energy needed to run in the process, there is a need to carefully control the reaction conditions, (e.g., to control the flow rates and reactor temperature dissipation) to avoid producing products with poor physical characteristics, such as off-odor and/or color. [See for example, pages 213-214 of "Sulfonation Technology in the Detergent Industry", ibid]
More specifically, side reactions that may occur, at least in part owing to conventional temperatures and the vigor of the sulfating agent include: dehydration of alkyl sulfate to form olefin; elimination of water between two molecules to form an ether; oxidation to the aldehyde followed by further oxidation to the acid; and esterification of the acid with fatty alcohol [see: David D. Whyte, J.A.O.C.S., Vol. 32, pp313-316, (1955)].

Thus, milder conditions for such longer chain alcohols is highly sought after in the industry. It has been discovered by the present invention that inclusion of at least 10% of a mid-chain branched, longer chain alcohol mixture into the longer chain length (at least about C_{14.5} average) alcohol mixture to be sulfated permits avoidance of high peak instantaneous reaction temperatures of the sulfation reaction, sufficiently to greatly improves the physical characteristics of the longer chain alcohol sulfate thereby produced. Similar benefits can also be obtained for the production of alkyl alkoxylated sulfate surfactant compositions.

Objects of the present invention therefore include one or more of the following: better economy by virtue of lower energy input in preparing the alcohol for the sulfation process and the ability to avoid having to melt out and control the feed alcohol temperature; faster reaction times; the ability to provide a more mass-effective product, by avoiding the need to include diluents and viscosity reducers; better color compared with high-temperature processing, such as required for tallow alcohols, and thereby avoiding the need for a subsequent bleaching operation; lower viscosity of the "acid mix" (the term used to describe the product of sulfation prior to being neutralized), which in turn improves the ease and quality of the neutralization step; lower viscosity of the finished surfactant paste at identical temperature compared to the process required for the tallow alcohol analog, which thereby permits the manufacture of more concentrated paste for use in so called "compact" or "ultra" detergent products, the ability to reduce or eliminate alcohol or similar materials which are frequently used to thin the final paste; the ability to manufacture new alkyl sulfate products which are completely linear alkyl benzene sulfonate-free (typical current practice is to co-react tallow alcohol and linear alkyl benzene to make a mixed tallow alcohol sulfate and linear alkyl benzene sulfonate product); the ability to more easily pump and handle all materials in the sulfation process from starting material to final product; the ability to avoid or eliminate hot storage steps, and the associated costs and potential discoloration of product that may thereby be produced; the ability to use relatively low-cost sulfonation plants which under normal circumstances would be intolerant of, or could not handle, neat C₁₆₋₁₈ alcohols; and the ability to limit side-reactions during sulfation, thereby giving a cleaner product. These and other objects of the present invention will be apparent from the detailed description hereinafter.

### BACKGROUND ART

See: Kirk Othmer, Encyclopedia of Chemical Technology, 3rd. Ed., Wiley, 1983, Vol. 22, pp 28-45, article entitled "Sulfonation and Sulfation"; W.H. De Groot, "sulphonation Technology in the Detergent Industry", Kluwer Academic Publishers, Boston, 1991; ISBN 0-7923-1202-3; and "Detergent Manufacturing Including Zeolite Builders and Other New Materials,", Chemical Technology Review No. 128, Noyes Data Corp., Park Ridge, New Jersey. 1979, pp. 273-310.

### SUMMARY OF THE INVENTION

The present invention relates to a method for manufacturing longer chain alkyl sulfate surfactant mixture compositions, said method comprising the steps of:
(a) sulfating with SO₃ (preferably in a falling film reactor) a long chain aliphatic alcohol mixture having an average carbon chain length of at least 14.5 to about 17.5, said alcohol mixture comprising at least about 10 % (preferably at least about 25%, more preferably at least about 50%, still more preferably at least about 75%, and most preferably at least about 95%) of a mid-chain branched aliphatic alcohol having the formula: 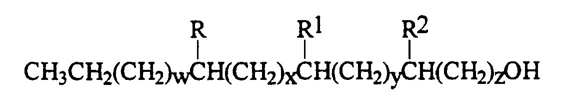 wherein the total number of carbon atoms in the branched primary alkyl moiety of this formula (including the R, R¹, and R² branching) is from 14 to 20, and wherein further for this alcohol mixture the average total number of carbon atoms in the branched primary alkyl moieties having the above formula is within the range of greater than 14.5 to about 17.5 (preferably greater than about 15 to about 17); R, R¹, and R² are each independently selected from hydrogen and C₁-C₃ alkyl (preferably methyl), provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; and w + x + y + z is from 8 to 14; and
(b) neutralizing the alkyl sulfate acid produced by step (a).

Preferred processes further utilize an alcohol feed temperature for a step (a) of less than about 50°C, preferably within the range of from about 10°C to about 50°C, more preferably within the range of from about 15°C to about 45°C, and most preferably from about 20°C to about 35°C: Typical conditions also include: a mole ratio of SO₃ to alcohol slightly greater than about 1:1; SO₃ concentrations in the range of 4-5%, volume/volume, in air or nitrogen (although no-air processes can also be used); cooling jacket water temperatures in the range of from about 20°C to about 30°C; neutralization temperatures within the range of from about 25°C to about 35°C; acid feed temperature into the neutralizer within the range of from about 35°C to about 45°C. It is also preferred that the final alkyl sulfate paste concentration be greater than about 20%, preferably greater than about 50%, and most preferably greater than about 60%.

Preferably, the aliphatic alcohol used in the above process comprises at least about 10% (preferably at least about 25%, more preferably at least about 50%, still more preferably at least about 75%, and most preferably at least about 95%) by weight of a mixture of two or more mid-chain branched alcohols having the formula: 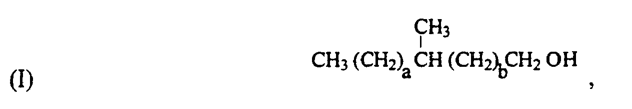 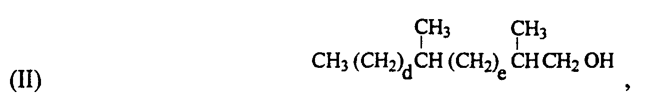 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14; a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;
wherein for this alcohol mixture the average total number of carbon atoms in the branched primary alkyl moieties having the above formulas is within the range of greater than 14.5 to about 17.5.

Optionally, the present invention process may include a first alkoxylation step whereby a long chain mid-chain branched polyoxyalkylene alcohol compound is produced, which thereafter is sulfated to produce alkyl alkoxylated sulfates according to the present invention. Such processes comprise the steps of:
(a) alkoxylating a long chain mid-chain branched aliphatic alcohol mixture having an average carbon chain length of at least 14.5 to about 17.5, said alcohol mixture comprising at least about 10% (preferably at least about 25%, more preferably at least about 50%, still more preferably at least about 75%, and most preferably at least about 95%) of a mid-chain branched aliphatic alcohol having the formula: 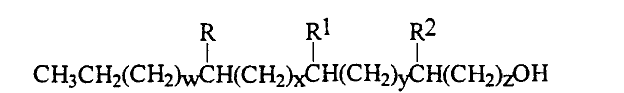 wherein the total number of carbon atoms in the branched primary alkyl moiety of this formula (including the R, R¹, and R² branching) is from 14 to 20, and wherein further for this alcohol mixture the average total number of carbon atoms in the branched primary alkyl moieties having the above formula is within the range of greater than 14.5 to about 17.5 (preferably greater than about 15 to about 17); R, R¹, and R² are each independently selected from hydrogen and C₁-C₃ alkyl (preferably methyl), provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; and w + x + y + z is from 8 to 14;
(b) sulfating the polyoxyalkylene alcohol produced by step (a) with SO₃; and
(c) neutralizing the alkyl alkoxylate sulfate acid produced by step (b).

The present invention therefore also may produce alkyl alkoxylated sulfates directly starting from a polyoxyalkylene alcohol. Such method comprises the steps of:
(a) sulfating with SO₃ a long chain aliphatic polyoxyalkylene alcohol mixture having an average aliphatic alcohol carbon chain length of at least 14.5 to about 17.5, said polyoxyalkylene alcohol mixture comprising at least about 10% (preferably at least about 25%, more preferably at least about 50%, still more preferably at least about 75%, and most preferably at least about 95%) of a mid-chain branched aliphatic polyoxyalkylene alcohol having the formula: 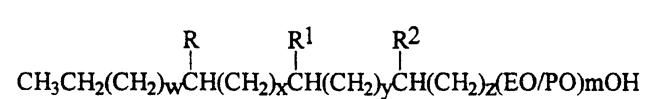 wherein the total number of carbon atoms in the branched primary alkyl moiety of this formula (including the R, R¹, and R³ branching, but not including the carbon atoms in the EO/PO alkoxy moiety) is from 14 to 20, and wherein further for this polyoxyalkylene alcohol mixture the average total number of carbon atoms in the branched primary alkyl moieties having the above formula is within the range of greater than 14.5 to about 17.5 (preferably from about 15 to about 17); R, R¹, and R² are each independently selected from hydrogen and C₁-C₃ alkyl (preferably methyl), provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; w + x + y + z is from 8 to 14; and EO/PO are alkoxy moieties including for example ethoxy, propoxy, butoxy, etc. preferably selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5 [it is to be recognized that the (EO/PO)ₘ moiety may be either a distribution with average degree of alkoxylation corresponding to m, or it may be a single specific chain with alkoxylation (e.g., ethoxylation and/or propoxylation) of exactly the number of units corresponding to m]; and
(b) neutralizing the alkyl alkoxylated sulfate acid produced by step (a).

Preferably, the aliphatic polyoxyalkylene alcohol used in step (a) of this process comprises at least about 10% (preferably at least about 25%, more preferably at least about 50%, still more preferably at least about 75%, and most preferably at least about 95%) by weight of a mixture of two or more mid-chain branched aliphatic polyoxyalkylene alcohols having the formula: 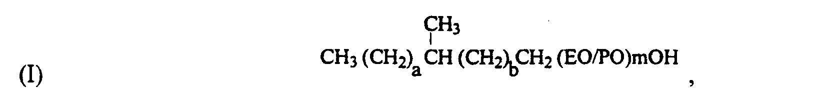 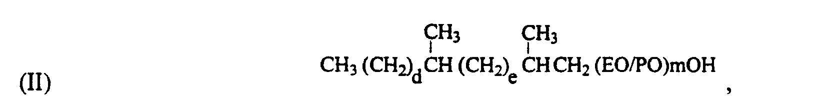 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from I to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;

wherein for this polyoxyalkylene alcohol mixture the average total number of carbon atoms in the branched primary alkyl moieties having the above formulas is within the range of greater than 14.5 to about 17.5; and
wherein EO/PO are alkoxy moieties, preferably selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5.

All percentages, ratios and proportions herein are by weight, unless otherwise specified. All temperatures are in degrees Celsius (°C) unless otherwise specified. All documents cited are in relevant part, incorporated herein by reference.

### DETAILED DESCRIPTION OF THE INVENTION

The present invention sulfation process may start with alcohols and/or polyoxyalkylene alcohols essentially comprising at least about 10% (preferably at least about 25%, more preferably at least about 50%, still more preferably at least about 75%, and most preferably at least about 95%) by weight of the following mid-chain branched materials.

### (a) Mid-chain branched aliphatic alcohols:

Mid-chain branched aliphatic alcohols useful in the present invention sulfation process have the formula:

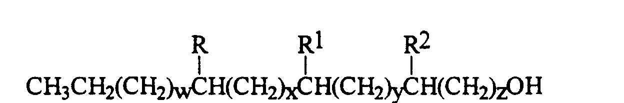

The alcohol mixtures useful in the present invention comprise molecules having a linear primary alkyl chain backbone (i.e., the longest linear carbon chain which includes the hydroxylated carbon atom). These alkyl chain backbones comprise from 12 to 19 carbon atoms; and further the molecules comprise a branched primary alkyl moiety having at least a total of 14, but not more than 20, carbon atoms. In addition, the alcohol mixture has an average total number of carbon atoms for the branched primary alkyl moieties within the range of from greater than 14.5 to about 17.5. Thus, the present invention mixtures comprise at least one branched primary alkyl alcohol compound having a longest linear carbon chain of not less than 12 carbon atoms or more than 19 carbon atoms, and the total number of carbon atoms including branching must be at least 14, and further the average total number of carbon atoms for the branched primary alkyl chains is within the range of greater than 14.5 to about 17.5.

For example, a C16 total carbon primary alkyl alcohol having 13 carbon atoms in the backbone must have 1, 2, or 3 branching units (i.e., R, R¹ and/or R³) whereby total number of carbon atoms in the molecule is at least 16. In this example, the C16 total carbon requirement may be satisfied equally by having, for example, one propyl branching unit or three methyl branching units.

R, R¹, and R² are each independently selected from hydrogen and C₁-C₃ alkyl (preferably hydrogen or C₁-C₂ alkyl, more preferably hydrogen or methyl, and most preferably methyl), provided R, R¹, and R² are not all hydrogen. Further, when z is 1, at least R or R¹ is not hydrogen.

Although for the purposes of the present invention alcohol compositions having the above formula do not include molecules wherein the units R, R¹, and R² are all hydrogen (i.e., linear non-branched primary alkyl sulfates), it is to be recognized that the present invention compositions may still further comprise some amount of linear, non-branched primary alkyl alcohol. Further, this linear non-branched primary alkyl alcohol surfactant may be present as the result of the process used to manufacture the alcohol mixture having the requisite one or more mid-chain branched primary alkyl sulfates according to the present invention, or for purposes of formulating detergent compositions some amount of linear non-branched primary alkyl alcohol may be admixed into the alcohol mixture.

Further regarding the above formula, w is an integer from 0 to 13; x is an integer from 0 to 13; y is an integer from 0 to 13; z is an integer of at least 1; and w + x + y + z is an integer from 8 to 14.

Certain points of branching (i.e., the location along the chain of the R, R¹, and/or R² moieties in the above formula) are preferred over other points of branching along the backbone of the alcohol. The formula below illustrates the mid-chain branching range (i.e., where points of branching occur), preferred mid-chain branching range, and more preferred mid-chain branching range for mono-methyl substituted linear alkyl alcohols (the same applies to the polyoxyalkylene alcohols described in detail herein after) of the present invention. 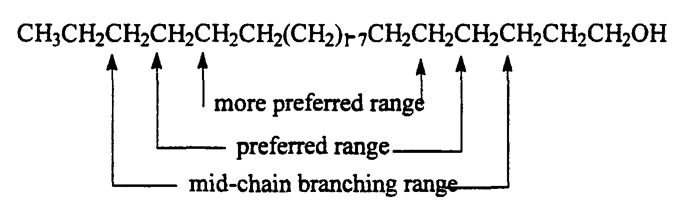 It should be noted that for the mono-methyl substituted alcohols these ranges exclude the two terminal carbon atoms of the chain and the two carbon atoms immediately adjacent to the hydroxyl (or polyoxyalkylene moieties) group. For alcohol mixtures comprising two or more of R, R¹, or R², alkyl branching at the 2-carbon atom is within the scope of the present invention. Alcohols having chains longer than ethyl (i.e. C₃ alkyl substitutents) on the 2-carbon atom, however, are less preferred.

The formula below illustrates the mid-chain branching range, preferred mid-chain branching range, and more preferred mid-chain branching range for di-methyl substituted linear alkyl alcohols (the same applies to the polyoxyalkylene alcohols described in detail herein after) of the present invention. 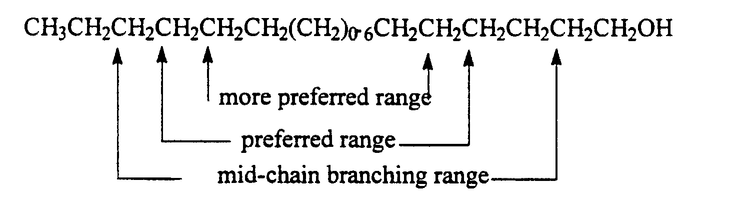 When di-alkyl substituted primary alkyl alcohols are combined with monosubstituted mid-chain branched primary alkyl alcohols, the di-alkyl substituted primary alkyl alcohols having one methyl substitution on the 2-carbon position and another methyl substitution in the preferred range as indicated above, are within the present invention.

The preferred alcohol mixtures of the present invention have at least 0.001%, more preferably at least 5%, most preferably at least 20% by weight, of the mixture one or more branched primary alkyl sulfates having the formula 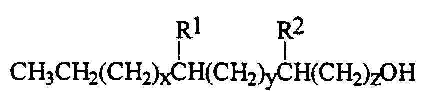 wherein the total number of carbon atoms, including branching, is from 15 to 18, and wherein further for this alcohol mixture the average total number of carbon atoms in the branched primary alkyl moieties having the above formula is within the range-of greater than 14.5 to about 17.5; R¹ and R² are each independently hydrogen or C₁-C₃ alkyl; x is from 0 to 11; y is from 0 to 11; z is at least 2; and x + y + z is from 9 to 13; provided R¹ and R² are not both hydrogen. More preferred are compositions having at least 5% of the mixture comprising one or more mid-chain branched primary alkyl sulfates wherein x + y is equal to 9 and z is at least 2.

Preferably, the mixtures of alcohols comprise at least 5% of a mid chain branched primary alkyl alcohol having R¹ and R² independently hydrogen, methyl, provided R¹ and R² are not both hydrogen; x + y is equal to 8, 9, or 10 and z is at least 2. More preferably the mixtures of alcohol comprise at least 20% of a mid chain branched primary alkyl alcohol having R¹ and R² independently hydrogen, methyl, provided R¹ and R² are not both hydrogen; x + y is equal to 8,9, or 10 and z is at least 2.

Preferred alcohol mixtures according to the present invention comprise from about 0.001% to about 99% of a mixture of mid-chain branched primary alkyl alcohols, said mixture comprising at least about 5 % by weight of two or more mid-chain branched alkyl alcohols having the formula: 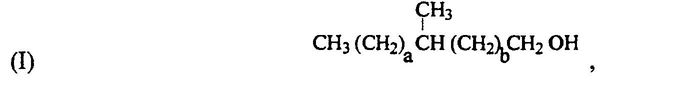 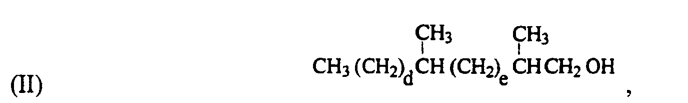 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from I to 11;
when a + b = 14, a is an integer from 2 to 13 and b is an integer from I to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;
wherein further for this surfactant mixture the average total number of carbon atoms in the branched primary alkyl moieties having the above formulas is within the range of greater than 14.5 to about 17.5.

Preferred mono-methyl branched primary alkyl alcohols useful in the present process are selected from the group consisting of: 3-methyl pentadecanol , 4-methyl pentadecanol , 5-methyl pentadecanol , 6-methyl pentadecanol, 7-methyl pentadecanol , 8-methyl pentadecanol , 9-methyl pentadecanol, 10-methyl pentadecanol, 11-methyl pentadecanol, 12-methyl pentadecanol, 13-methyl pentadecanol, 3-methyl hexadecanol , 4-methyl hexadecanol, 5-methyl hexadecanol , 6-methyl hexadecanol , 7-methyl hexadecanol , 8-methyl hexadecanol , 9-methyl hexadecanol, 10-methyl hexadecanol, 11-methyl hexadecanol, 12-methyl hexadecanol, 13-methyl hexadecanol, 14-methyl hexadecanol, and mixtures thereof.

Preferred di-methyl branched primary alkyl alcohols are selected from the group consisting of: 2,3-methyl tetradecanol , 2,4-methyl tetradecanol, 2,5-methyl tetradecanol , 2,6-methyl tetradecanol , 2,7-methyl tetradecanol , 2,8-methyl tetradecanol , 2,9-methyl tetradecanol , 2,10-methyl tetradecanol , 2,11-methyl tetradecanol , 2,12-methyl tetradecanol , 2,3-methyl pentadecanol , 2,4-methyl pentadecanol , 2,5-methyl pentadecanol , 2,6-methyl pentadecanol , 2,7-methyl pentadecanol , 2,8-methyl pentadecanol , 2,9-methyl pentadecanol , 2,10-methyl pentadecanol , 2,11-methyl pentadecanol , 2,12-methyl pentadecanol , 2,13-methyl pentadecanol , and mixtures thereof.

### (b) Mid-chain branched aliphatic polyoxyalkylene alcohols:

Mid-chain branched aliphatic polyoxyalkylene alcohols useful in the present invention sulfation process have the formula:

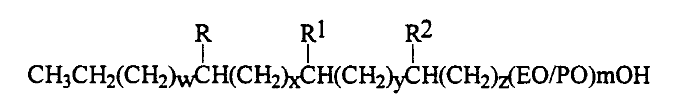

These alcohol mixtures of the present invention comprise molecules having a linear primary polyoxyalkylene chain backbone (i.e., the longest linear carbon chain which includes the alkoxylated carbon atom). These alkyl chain backbones comprise from 12 to 19 carbon atoms; and further the molecules comprise a branched primary alkyl moiety having at least a total of 14, but not more than 20, carbon atoms. In addition, the alcohol mixture has an average total number of carbon atoms for the branched primary alkyl moieties within the range of from greater than 14.5 to about 17.5. Thus, the present invention mixtures comprise at least_one polyoxyalkylene compound having a longest linear carbon chain of not less than 12 carbon atoms or more than 19 carbon atoms, and the total number of carbon atoms including branching must be at least 14, and further the average total number of carbon atoms for the branched primary alkyl chains is within the range of greater than 14.5 to about 17.5.

For example, a C16 total carbon (in the alkyl chain) primary polyoxyalkylene alcohol having 15 carbon atoms in the backbone must have a methyl branching unit (either R, R¹ or R² is methyl) whereby the total number of carbon atoms in the molecule is 16.

R, R¹, and R² are each independently selected from hydrogen and C₁-C₃ alkyl (preferably hydrogen or C₁-C₂ alkyl, more preferably hydrogen or methyl, and most preferably methyl), provided R, R¹, and R² are not all hydrogen. Further, when z is 1, at least R or R¹ is not hydrogen.

Although for the purposes of the present invention alcohol compositions the above formula does not include molecules wherein the units R, R¹, and R² are all hydrogen (i.e., linear non-branched primary polyoxyalkylenes), it is to be recognized that the present invention compositions may still further comprise some amount of linear, non-branched primary polyoxyalkylene. Further, this linear non-branched primary polyoxyalkylene alcohol may be present as the result of the process used to manufacture the alcohol mixture having the requisite mid-chain branched primary polyoxyalkylenes according to the present invention, or for purposes of formulating detergent compositions some amount of linear non-branched primary polyoxyalkylene may be admixed into the final product formulation.

Further it is to be similarly recognized that non-alkoxylated mid-chain branched alcohol may comprise some amount of the present invention polyoxyalkylene-containing compositions. Such materials may be present as the result of incomplete alkoxylation of the alcohol used to prepare the polyoxyalkylene alcohol, or these alcohols may be separately added to the present invention alcohol mixtures along with a mid-chain branched polyoxyalkylene alcohol according to the present invention.

Further regarding the above formula, w is an integer from 0 to 13; x is an integer from 0 to 13; y is an integer from 0 to 13; z is an integer of at least 1; and w + x + y + z is an integer from 8 to 14.

EO/PO are alkoxy moieties, preferably selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5. The (EO/PO)ₘ moiety may be either a distribution with average degree of alkoxylation (e.g., ethoxylation and/or propoxylation) corresponding to m, or it may be a single specific chain with alkoxylation (e.g., ethoxylation and/or propoxylation) of exactly the number of units corresponding to m.

The preferred alcohol mixtures of the present invention have at least 0.001%, more preferably at least 5%, most preferably at least 20% by weight, of the mixture one or more mid-chain branched primary alkyl polyoxyalkylenes having the formula 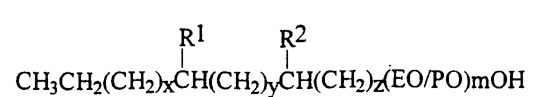 wherein the total number of carbon atoms, including branching, is from 15 to 18, and wherein further for this alcohol mixture the average total number of carbon atoms in the branched primary alkyl moieties having the above formula is within the range of greater than 14.5 to about 17.5; R¹ and R² are each independently hydrogen or C₁-C₃ alkyl; x is from 0 to 11; y is from 0 to 11; z is at least 2; and x + y + z is from 9 to 13; provided R¹ and R² are not both hydrogen; and EO/PO are alkoxy moieties selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5. More preferred are compositions having at least 5% of the mixture comprising one or more mid-chain branched primary polyoxyalkylenes wherein z is at least 2.

Preferably, the mixtures of surfactant comprise at least 5%, preferably at least about 20%, of a mid chain branched primary alkyl polyoxyalkylene having R¹ and R² independently hydrogen or methyl, provided R¹ and R² are not both hydrogen; x + y is equal to 8, 9 or 10 and z is at least 2.

Preferred alcohol mixtures according to the present invention comprise from about 0.001% to about 99% of a mixture of mid-chain branched primary alkyl polyoxyalkylene alcohols, said mixture comprising at least about 5 % by weight of one or more mid-chain branched alkyl polyoxyalkylenes having the formula: 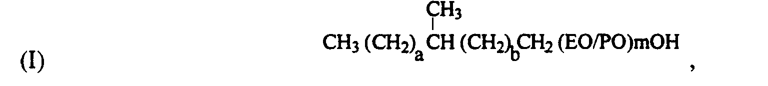 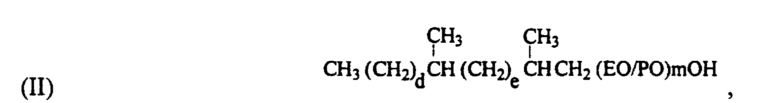 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from I to 11;
when d + e = 14, d is an integer from 2 to 13 and e is an integer from 1 to 12;
and wherein further for this alcohol mixture the average total number of carbon atoms in the branched primary alkyl moieties having the above formulas is within the range of greater than 14.5 to about 17.5; and EO/PO are alkoxy moieties selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5.

Preferred mono-methyl branched primary alkyl ethoxylates are selected from the group consisting of: 3-methyl pentadecanol ethoxylate, 4-methyl pentadecanol ethoxylate, 5-methyl pentadecanol ethoxylate, 6-methyl pentadecanol ethoxylate, 7-methyl pentadecanol ethoxylate, 8-methyl pentadecanol ethoxylate, 9-methyl pentadecanol ethoxylate, 10-methyl pentadecanol ethoxylate, 11-methyl pentadecanol ethoxylate, 12-methyl pentadecanol ethoxylate, 13-methyl pentadecanol ethoxylate, 3-methyl hexadecanol ethoxylate, 4-methyl hexadecanol ethoxylate, 5-methyl hexadecanol ethoxylate, 6-methyl hexadecanol ethoxylate, 7-methyl hexadecanol ethoxylate, 8-methyl hexadecanol ethoxylate, 9-methyl hexadecanol ethoxylate, 10-methyl hexadecanol ethoxylate, 11-methyl hexadecanol ethoxylate, 12-methyl hexadecanol ethoxylate, 13-methyl hexadecanol ethoxylate, 14-methyl hexadecanol ethoxylate, and mixtures thereof, wherein the compounds are ethoxylated with an average degree of ethoxylation of from about 0.1 to about 10.

Preferred di-methyl branched primary alkyl ethoxylates selected from the group consisting of: 2,3-methyl tetradecanol ethoxylate, 2,4-methyl tetradecanol ethoxylate, 2,5-methyl tetradecanol ethoxylate, 2,6-methyl tetradecanol ethoxylate, 2,7-methyl tetradecanol ethoxylate, 2,8-methyl tetradecanol ethoxylate, 2,9-methyl tetradecanol ethoxylate, 2,10-methyl tetradecanol ethoxylate, 2,11-methyl tetradecanol ethoxylate, 2,12-methyl tetradecanol ethoxylate, 2,3-methyl pentadecanol ethoxylate, 2,4-methyl pentadecanol ethoxylate, 2,5-methyl pentadecanol ethoxylate, 2,6-methyl pentadecanol ethoxylate, 2,7-methyl pentadecanol ethoxylate, 2,8-methyl pentadecanol ethoxylate, 2,9-methyl pentadecanol ethoxylate, 2,10-methyl pentadecanol ethoxylate, 2,11-methyl pentadecanol ethoxylate, 2,12-methyl pentadecanol ethoxylate, 2,13-methyl pentadecanol ethoxylate, and mixtures thereof, wherein the compounds are ethoxylated with an average degree of ethoxylation of from about 0.1 to about 10.

### Sulfation Conditions:

The sulfation process according to the present invention utilizes SO₃ as the sulfating reagent. SO₃ concentrations in the range of 4-5%, volume/volume, in air are preferred, although no-air processes can also be used. A slight molar excess of SO₃, i.e., a mole ratio of SO₃ to alcohol slightly greater than about 1:1, is preferred.

Preferred processes further utilize an alcohol feed temperature for the sulfation step of the process of less than about 50°C, preferably within the range of from about 10°C to about 50°C, more preferably within the range of from about 15°C to about 45°C, and most preferably from about 20°C to about 35°C. Cooling jacket water temperatures in the range of from about 20°C to about 30°C are typical for the present process.

The processes herein may utilize continuous or batch reactors. Preferred process utilize continuous reactors such as a falling film reactor in which to react the alcohol and the SO₃. Specific reactors include Chemithon, Ballestra, and other reactors as described in: Kirk Othmer, Encyclopedia of Chemical Technology, 3rd. Ed., Wiley, 1983, Vol. 22, pp 28-45, article entitled "Sulfonation and Sulfation"; W.H. De Groot, "sulphonation Technology in the Detergent Industry", Kluwer Academic Publishers, Boston, 1991; ISBN 0-7923-1202-3; and "Detergent Manufacturing Including Zeolite Builders and Other New Materials,", Chemical Technology Review No. 128, Noyes Data Corp., Park Ridge, New Jersey, 1979, pp. 273-310.

### Neutralization:

The step of Neutralizing the alkyl sulfate acid or alkyl alkoxylated sulfate acid produced by the sulfation reaction is preferably conducted immediately after the sulfation reaction. Typical conditions for this step of the process include neutralization temperatures within the range of from about 25°C to about 35°C, and acid feed temperatures into the neutralizer within the range of from about 35°C to about 45°C.

Basic neutralizing agents useful herein include any suitable inorganic base. For example, potassium hydroxide, sodium hydroxide, ammonia monoethanolamine, and triethanolamine, may be used. Potassium salts may be useful for further reduction in viscosity of aqueous surfactant pastes.

It is also preferred that the final alkyl sulfate paste concentration which results from the neutralization step be greater than about 20%, preferably greater than about 50%, and most preferably greater than about 60%.

As a result of the present process, alkyl sulfate and alkyl alkoxylated sulfate surfactants are produced comprising mid-chain branched primary alkyl sulfates and/or mid-chain primary alkyl alkoxylated sulfates having the following formulas.

The present invention produces surfactant compositions comprising mid-chain branched primary alkyl sulfates having the formula: 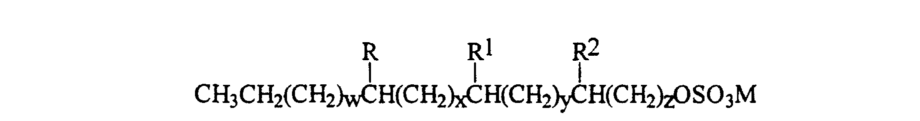 wherein the total number of carbon atoms in the branched primary alkyl moiety of this formula (including the R, R¹, and R² branching) is from 14 to 20, and wherein further for this surfactant mixture the average total number of carbon atoms in the branched primary alkyl moieties having the above formula is within the range of greater than 14.5 to about 17.5 (preferably from about 15 to about 17); R, R¹, and R² are each independently selected from hydrogen and C₁-C₃ alkyl (preferably methyl), provided R, R¹, and R² are not all hydrogen and, when z is 1, at least R or R¹ is not hydrogen; M is one or more cations; w is an integer from 0 to 13; x is an integer from 0 to 13; y is an integer from 0 to 13; z is an integer of at least 1; and w + x + y + z is from 8 to 14. Preferably less than about 80% of the alkyl sulfates have a total of 18 carbon atoms in the alkyl chain.

The present invention also produces surfactant compositions comprising mid-chain branched primary alkyl alkoxylated sulfates having the formula: 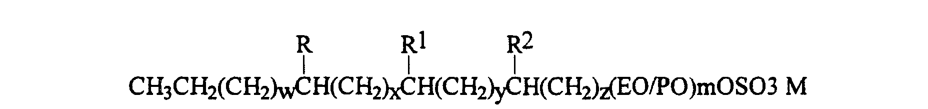 wherein the total number of carbon atoms in the branched primary alkyl moiety of this formula (including the R, R¹, and R³ branching, but not including the carbon atoms in the EO/PO alkoxy moiety) is from 14 to 20, and wherein further for this surfactant mixture the average total number of carbon atoms in the branched primary alkyl moieties having the above formula is within the range of greater than 14.5 to about 17.5 (preferably from about 15 to about 17); R, R¹, and R² are each independently selected from hydrogen and C₁-C₃ alkyl (preferably methyl), provided R, R¹, and R² are not all hydrogen and, when z is 1, at least R or R¹ is not hydrogen; M is one or more cations; w is an integer from 0 to 13; x is an integer from 0 to 13; y is an integer from 0 to 13; z is an integer of at least 1; w + x + y + z is from 8 to 14; and EO/PO are alkoxy moieties including for example ethoxy, propoxy, butoxy, etc, preferably selected from ethoxy, propoxy, and mixed ethoxy/propoxy groups, wherein m is at least about 0.01, preferably within the range of from about 0.1 to about 30, more preferably from about 0.5 to about 10, and most preferably from about 1 to about 5. It is to be recognized that the (EO/PO)ₘ moiety may be either a distribution with average degree of alkoxylation corresponding to m, or it may be a single specific chain with alkoxylation (e.g., ethoxylation and/or propoxylation) of exactly the number of units corresponding to m.

As used herein, M is a salt forming cation depending upon the method of synthesis. Examples of salt forming cations are lithium, sodium, potassium, calcium, magnesium, quaternary alkyl amines having the formula 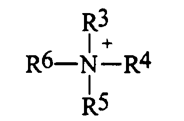 wherein R³, R⁴, R⁵ and R⁶ are independently hydrogen, C₁-C₂₂ alkylene, C₄-C₂₂ branched alkylene, C₁-C₆ alkanol, C₁-C₂₂ alkenylene, C₄-C₂₂ branched alkenylene, and mixtures thereof. Preferred cations are ammonium (R³, R⁴, R⁵ and R⁶ equal hydrogen), sodium, potassium, mono-, di-, and trialkanol ammonium, and mixtures thereof. The monoalkanol ammonium compounds of the present invention have R³ equal to C₁-C₆ alkanol, R⁴, R⁵ and R⁶ equal to hydrogen; dialkanol ammonium compounds of the present invention have R³ and R⁴ equal to C₁-C₆ alkanol, R⁵ and R⁶ equal to hydrogen; trialkanol ammonium compounds of the present invention have R³, R⁴ and R⁵ equal to C₁-C₆ alkanol, R⁶ equal to hydrogen. Preferred alkanol ammonium salts of the present invention are the mono-, di- and tri- quaternary ammonium compounds having the formulas:

H₃N⁺CH₂CH₂OH, H₂N⁺(CH₂CH₂OH)₂, HN⁺(CH₂CH₂OH)₃.

Preferred M is sodium, potassium and the C₂ alkanol ammonium salts listed above; most preferred is sodium.

### Preparation of Mid-chain Branched Alcohols and Polyoxyalkylene Alcohols

The following reaction scheme outlines a general approach to the preparation of the mid-chain branched primary alcohols useful for the present invention process.

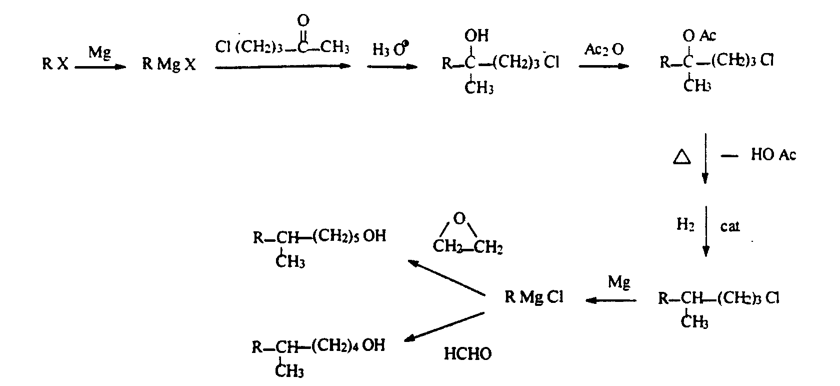

An alkyl halide is converted to a Grignard reagent and the Grignard is reacted with a haloketone. After conventional acid hydrolysis, acetylation and thermal elimination of acetic acid, an intermediate olefin is produced (not shown in the scheme) which is hydrogenated forthwith using any convenient hydrogenation catalyst such as Pd/C.

This route is favorable over others in that the branch, in this illustration a 5-methyl branch, is introduced early in the reaction sequence.

Formylation of the alkyl halide resulting from the first hydrogenation step yields alcohol product, as shown in the scheme. This may be alkoxylated using standard techniques to yield a mid-chain branched primary alkyl polyoxyalkylene alcohol. There is flexibility to extend the branching one additional carbon beyond that which is achieved by a single formylation. Such extension can, for example, be accomplished by reaction with ethylene oxide. See "Grignard Reactions of Nonmetallic Substances", M.S. Kharasch and O. Reinmuth, Prentice-Hall, N.Y., 1954; *J*. *Org*. *Chem.,* J. Cason and W. R. Winans, Vol. 15 (1950), pp 139-147; *J*. *Org Chem.,* J. Cason et al., Vol. 13 (1948), pp 239-248; J. Org *Chem*., J. Cason et al., Vol. 14 (1949), pp 147-154; and *J*. *Org Chem.,* J. Cason et al., Vol. 15 (1950), pp 135- 138 all of which are incorporated herein by reference.

In variations of the above procedure, alternate haloketones or Grignard reagents may be used. PBr3 halogenation of the alcohol from formylation or ethoxylation can be used to accomplish an iterative chain extension.

The preferred mid-chained branched primary alkyl alcohols and therefrom the preferred polyoxyalkylene alcohols useful in the present invention can also be readily prepared as follows:

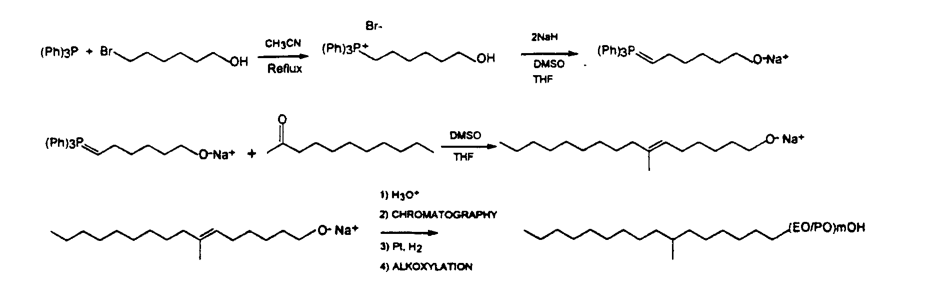

A conventional bromoalcohol is reacted with triphenylphosphine followed by sodium hydride, suitably in dimethylsulfoxide/tetrahydrofuran, to form a Wittig adduct. The Wittig adduct is reacted with an alpha methyl ketone, forming an internally unsaturated methyl-branched alcoholate. Hydrogenation, optionally followed by alkoxylation, yields the desired mid-chain branched primary alkyl alcohols. Although the Wittig approach does not allow the practitioner to extend the hydrocarbon chain, as in the Grignard sequence, the Wittig typically affords higher yields. See *Agricultural and Biological Chemistry*, *M.* Horiike et al., vol. 42 (1978), pp 1963-1965 included herein by reference.

Any alternative synthetic procedure in accordance with the invention may be used to prepare the branched primary alkyl alcohols. The mid-chain branched primary alkyl alcohols may, in addition be synthesized or formulated in the presence of the conventional homologs, for example any of those which may be formed in an industrial process which produces 2-alkyl branching as a result of hydroformylation.

In certain preferred embodiments of the alcohol mixtures of the present invention, especially those derived from fossil fuel sources involving commercial processes, comprise at least 1 mid-chain branched primary alkyl alcohol, preferably at least 2, more preferably at least 5, most preferably at least 8.

Particularly suitable for preparation of certain alcohol mixtures for the present invention are "oxo" reactions wherein a branched chain olefin is subjected to catalytic isomerization and hydroformylation. The preferred processes resulting in such mixtures utilize fossil fuels as the starting material feedstock. Preferred processes utilize Oxo reaction on linear olefins (alpha or internal) with a limited amount of branching. Suitable olefins may be made by dimerization of linear alpha or internal olefins, by controlled oligomerization of low molecular weight linear olefins, by skeletal rearrangement of detergent range olefins, by dehydrogenation/skeletal rearrangement of detergent range paraffins, or by Fischer-Tropsch reaction. These reactions will in general be controlled to:
1) give a large proportion of olefins in the desired detergent range (while allowing for the addition of a carbon atom in the subsequent Oxo reaction),
2) produce a limited number of branches, preferably mid-chain,
3) produce C₁-C₃ branches, more preferably ethyl, most preferably methyl,
4) limit or eliminate gem dialkyl branching i.e. to avoid formation of quaternary carbon atoms. The suitable olefins can undergo Oxo reaction to give primary alcohols either directly or indirectly through the corresonding aldehydes. When an internal olefin is used, an Oxo catalyst is normally used which is capable of prior pre-isomerization of internal olefins primarily to alpha olefins. While a separately catalyzed (i.e. non-Oxo) internal to alpha isomerization could be effected, this is optional. On the other hand, if the olefin-forming step itself results directly in an alpha olefin (e.g. with high pressure Fischer-Tropsch olefins of detergent range), then use of a non-isomerizing Oxo catalyst is not only possible, but preferred.

The process described herein above gives the more preferred 5-methylhexadecyl alcohols in higher yield than the less preferred 2,4-dimethylpentadecyl alcohols. This mixture is desirable under the metes and bounds of the present invention in that each product comprises at total of 17 carbon atoms with linear alkyl chains having at least 13 carbon atoms.

The following examples provide methods for synthesizing various compounds useful in the present invention processes.

### EXAMPLE I

### Preparation of 7-methylhexadecyl ethoxylate (E2)

### Synthesis of (6-hydroxyhexyl) triphenylphosphonium bromide

Into a 5L, 3 neck round bottom flask fitted with nitrogen inlet, condenser, thermometer, mechanical stirring and nitrogen outlet is added 6-bromo-1-hexanol (500g, 2.76 mol), triphenylphosphine (768g, 2.9mol) and acetonitrile (1800 ml) under nitrogen. The reaction mixture is heated to reflux for 72 hrs. The reaction mixture is cooled to room temperature and transferred into a 5L beaker. The product is recrystallized from anhydrous ethyl ether (1.5L) at 10°C. Vacuum filtration followed by washing with ethyl ether and drying in a vacuum oven at 50°C for 2 hrs. gives 1140g of the desired product as white crystals.

### Synthesis of 7- methylhexadecene-1-ol

Into a dried 5L, 3 neck round bottom flask fitted with mechanical stirring, nitrogen inlet, dropping funnel, thermometer and nitrogen outlet is added 70.2g of 60% sodium hydride (1.76 mol) in mineral oil. The mineral oil is removed by washing with hexanes. Anhydrous dimethyl sulfoxide (500ml) is added to the flask and the mixture is heated to 70°C until evolution of hydrogen stops. The reaction mixture is cooled to room temperature followed by addition of 1L of anhydrous tetrahydrofuran. (6-hydroxyhexyl) triphenylphosphonium bromide (443.4g, 1 mol) is slurried with warm anhydrous dimethyl sulfoxide (50°C, 500ml) and slowly added to the reaction mixture through the dropping funnel while keeping it at 25-30°C. The mixture is stirred for 30 minutes at room temperature at which time 2-undecanone (187g, 1.1 mol) is slowly added through a dropping funnel. Reaction is slightly exothermic and cooling is needed to maintain 25-30°C. The mixture is stirred for 18 hr. and then poured into a 5L beaker containing 1L purified water with stirring. The oil phase (top) is allowed to separate out in a separatory funnel and the water phase is removed. The water phase is washed with hexanes (500ml) and the organic phase is separated and combined with the oil phase from the water wash. The organic mixture is then extracted with water 3 times (500ml each) followed by vacuum distillation to collect the clear, oily product (132g) at 140C and lmm Hg.

### Hydrogenation of 7- methylhexadecene-1-ol

Into a 3L rocking autoclave liner is added 7-methylhexadecene-1-ol (130g, 0.508mol), methanol (300ml) and platinum on carbon (10% by weight, 35g). The mixture is hydrogenated at 180°C under 1200 psig of hydrogen for 13 hrs., cooled and vacuum filtered thru Celite 545 with washing of the Celite 545, suitably with methylene chloride. If needed, the filtration can be repeated to eliminate traces of Pt catalyst, and magnesium sulfate can be used to dry the product. The solution of product is concentrated on a rotary evaporator to obtain a clear oil (124g).

### Alkoxylation of 7-methylhexadecanol

Into a dried 1L 3 neck round bottom flask fitted with a nitrogen inlet, mechanical stirrer, and a y-tube fitted with a thermometer and a gas outlet is added the alcohol from the preceeding step. For purposes of removing trace amounts of moisture, the alcohol is sparged with nitrogen for about 30 minutes at 80-100° C. Continuing with a nitrogen sweep, sodium metal is added as the catalyst and allowed to melt with stirring at 120-140° C. With vigorous stirring, ethylene oxide gas is added in 140 minutes while keeping the reaction temperature at 120-140° C. After the correct weight (equal to two equivalents of ethylene oxide) has been added, nitrogen is swept through the apparatus for 20-30 minutes as the sample is allowed to cool. The desired 7-methylhexadecyl ethoxylate (average of 2 ethoxylates per molecule) product is then collected.

### EXAMPLE II

### Synthesis of 7-methylpentadecyl ethoxylate (E3)

### Synthesis of (6-hydroxyhexyl) Triphenylphosphonium Bromide

Into a 5L, 3 neck round bottom flask fitted with nitrogen inlet, condenser, thermometer, mechanical stirring and nitrogen outlet is added 6-bromo-1-hexanol (500g, 2.76 mol), triphenylphosphine (768g, 2.9mol) and acetonitrile (1800 ml) under nitrogen. The reaction mixture is heated to reflux for 72 hrs. The reaction mixture is cooled to room temperature and transferred into a 5L beaker. The product is recrystallized from anhydrous ethyl ether (1.5L) at 10°C. Vacuum filtration of the mixture followed by washing the white crystals with ethyl ether and drying in a vacuum oven at 50°C for 2 hrs. gives 1140g of the desired product.

### Synthesis of 7- methylpentadecene-1-ol

Into a dried 5L, 3 neck round bottom flask fitted with mechanical stirring, nitrogen inlet, dropping funnel, thermometer and nitrogen outlet is added 80g of 60% sodium hydride (2.0 mol) in mineral oil. The mineral oil is removed by washing with hexanes. Anhydrous dimethyl sulfoxide (500ml) is added to the flask and heated to 70°C until evolution of hydrogen stops. The reaction mixture is cooled to room temperature followed by addition of 1L of anhydrous tetrahydrofuran. (6-hydroxyhexyl) triphenylphosphonium bromide (443.4g, 1 mol) is slurried with warm anhydrous dimethyl sulfoxide (50°C, 500ml) and slowly added to the reaction mixture thru the dropping funnel while keeping the reaction at 25-30°C. The reaction is stirred for 30 minutes at room temperature at which time 2-decanone (171.9g, 1.1 mol) is slowly added thru a dropping funnel. Reaction is slightly exothermic and cooling is needed to maintain 25-30°C. Mixture is stirred for 18 hrs. and then poured into a separatory funnel containing 600ml of purified water and 300 ml of hexanes. After shaking the oil phase (top) is allowed to separate out and the water phase is removed. The extractions of the oil phase are continued using water until both phases are clear. The organic phase is collected, vacuum distilled and purified by liquid chromatography (90:10 hexanes:ethyl acetate, silica gel stationary phase) to obtain a clear, oily product (119.1 g).

### Hydrogenation of 7- methylpentadecene-1-ol

Into a 3L rocking autoclave glass liner (Autoclave Engineers) is added 7-Methylpentadecene-1-ol (122g, 0.508mol), methanol (300ml) and platinum on carbon (10% by weight, 40g). The mixture is hydrogenated at 180°C under 1200 psig of hydrogen for 13 hrs., cooled and vacuum filtered thru Celite 545 with washing of Celite 545 with methylene chloride. The organic mixture is still dark from platinum catalyst so the filtration procedure is repeated with concentration on a rotary evaporator; dilution is carried out with methylene chloride (500ml) and magnesium sulfate is aded to dry product. Vacuum filter thru Celite 545 and concentrate filtrate on a rotary evaporator to obtain a clear oil (119g).

### Alkoxylation of 7-methylpentadecanol

Into a dried 1L 3 neck round bottom flask fitted with a nitrogen inlet, mechanical stirrer, and a y-tube fitted with a thermometer and a gas outlet is added the alcohol from the preceeding step. For purposes of removing trace amounts of moisture, the alcohol is sparged with nitrogen for about 30 minutes at 80-100° C. Continuing with a nitrogen sweep, sodium metal is added as the catalyst and allowed to melt with stirring at 120-140° C. With vigorous stirring, ethylene oxide gas is added in 140 minutes while keeping the reaction temperature at 120-140° C. After the correct weight (equal to three equivalents of ethylene oxide) has been added, nitrogen is swept through the apparatus for 20-30 minutes as the sample is allowed to cool. The desired 7-methylpentadecyl ethoxylate (average of 3 ethoxylates per molecule) product is then collected.

### EXAMPLE III

### Synthesis of 7-methylheptadecyl ethoxylate (E1.5)

### Synthesis of (6-Hydroxyhexyl) Triphenylphosphonium bromide

Into a 5L, 3 neck round bottom flask fitted with nitrogen inlet, condenser, thermometer, mechanical stirring and nitrogen outlet is added 6-bromo-1-hexanol (500g, 2.76 mol), triphenylphosphine (768g, 2.9mol) and acetonitrile (1800 ml) under nitrogen. The reaction mixture is heated to reflux for 72 hrs. The reaction mixture is cooled to room temperature and transferred into a 5L beaker. The product is recrystallized from anhydrous ethyl ether (1.5L) at 10°C. Vacuum filtration of the mixture followed by washing the white crystals with ethyl ether and drying in a vacuum oven at 50°C for 2 hrs. gives 1140g of the desired product.

### Synthesis of 7- methylheptadecene-1-ol

Into a dried 5L, 3 neck round bottom flask fitted with mechanical stirring, nitrogen inlet, dropping funnel, thermometer and nitrogen outlet is added 80g of 60% sodium hydride (2.0 mol) in mineral oil. The mineral oil is removed by washing with hexanes. Anhydrous dimethyl sulfoxide (500ml) is added to the flask and heated to 70°C until evolution of hydrogen stops. The reaction mixture is cooled to room temperature followed by addition of 1L of anhydrous tetrahydrofuran. (6-hydroxyhexyl) triphenylphosphonium bromide (443.4g, 1 mol) is slurried with warm anhydrous dimethyl sulfoxide (50°C, 500ml) and slowly added to the reaction mixture thru the dropping funnel while keeping the reaction at 25-30°C. The reaction is stirred for 30 minutes at room temperature at which time 2-dodecanone (184.3g, 1.1 mol) is slowly added thru a dropping funnel. Reaction is slightly exothermic and cooling is needed to maintain 25-30°C. Mixture is stirred for 18 hrs. and then poured into a separatory funnel containing 600ml of purified water and 300 ml of hexanes. After shaking the oil phase (top) is allowed to separate out and the water phase is removed which is cloudy. The extractions are continued using water until the water phase and the organic phase become clear. The organic phase is collected and purified by liquid chromatography (mobile phase-hexanes, stationary phase-silica gel ) to obtain a clear, oily product (116g). HNMR of the final product ( in deuterium oxide) indicates a CH₂-OSO₃⁻ triplet at the 3.8 ppm resonance, CH₂-CH₂-OSO₃⁻ multiplet at the 1.5 ppm resonance, CH₂ of the alkyl chain at the 0.9-1.3 ppm resonance and CH-CH₃ branch point overlapping the R-CH₂CH₃ terminal methyl group at the 0.8 ppm resonance.

### Hydrogenation of 7- methylheptadecene-1-ol

Into a 3L rocking autoclave glass liner (Autoclave Engineers) is added 7-Methylheptadecene-l-ol (116g, 0.433mol), methanol (300ml) and platinum on carbon (10% by weight, 40g). The mixture is hydrogenated at 180°C under 1200 psig of hydrogen for 13 hrs., cooled and vacuum filtered thru Celite 545 with washing of Celite 545 with methylene chloride. Vacuum filter thru Celite 545 and concentrate filtrate on a rotary evaporator to obtain a clear oil (108g).

### Alkoxylation of 7-methylheptadecanol

Into a dried 1L 3 neck round bottom flask fitted with a nitrogen inlet, mechanical stirrer, and a y-tube fitted with a thermometer and a gas outlet is added the alcohol from the preceeding step. For purposes of removing trace amounts of moisture, the alcohol is sparged with nitrogen for about 30 minutes at 80-100° C. Continuing with a nitrogen sweep, sodium metal is added as the catalyst and allowed to melt with stirring at 120-140° C. With vigorous stirring, ethylene oxide gas is added in 140 minutes while keeping the reaction temperature at 120-140° C. After the correct weight (equal to 1.5 equivalents of ethylene oxide) has been added, nitrogen is swept through the apparatus for 20-30 minutes as the sample is allowed to cool. The desired 7-methylheptadecyl ethoxylate (average of 1.5 ethoxylates per molecule) product is then collected.

### EXAMPLE IV

The following Shell Research experimental test alcohol samples are ethoxylated (average ethoxylation of 2.5) by the following procedure.

| ¹³C-NMR Results For Branched Alcohols Prepared | | | |
|---|---|---|---|
| Total Number of Carbons | 16 | 17 | 18 |
| Avg. Number of Branches per Molecule | 2.0 | 1.7 | 2.1 |
| | | | |

| Average Branch Position Relative To Hydroxyl Carbon | | | |
|---|---|---|---|
| % at C4 and higher | 56% | 55% | 52% |
| % at C3 | 26% | 21% | 25% |
| % at C2 | 18% | 24% | 23% |
| | | | |

| Type of Branching | | | |
|---|---|---|---|
| % propyl and higher | 31% | 35% | 30% |
| % ethyl | 12% | 10% | 12% |
| % methyl | 57% | 55% | 58% |

Into a dried 250ml 3 neck round bottom flask fitted with a nitrogen inlet, mechanical stirrer, and a y-tube fitted with a thermometer and a gas outlet is added the C16 alcohol (48.4g, 0.2 mol) above. For purposes of removing trace amounts of moisture, the alcohol is sparged with nitrogen for about 30 minutes at 80-100° C. Continuing with a nitrogen sweep, sodium metal (0.23g, 0.01 mol) is added as the catalyst and allowed to melt with stirring at 120-140° C. With vigorous stirring, ethylene oxide gas (22g, 0.5 mol) is added in 140 minutes while keeping the reaction temperature at 120-140° C. After the correct weight of ethylene oxide (average 2.5 ethoxylates per molecule) has been added, nitrogen is swept through the apparatus for 20-30 minutes as the sample is allowed to cool. The gold liquid product (69g, 0.196 mol) is bottled under nitrogen.

### EXAMPLE V

The alcohol of Example III (90 parts) is mixed with n-hexadecanol (10 parts). The starting alcohol mixture has a total carbonyl impurity level of no more than about 5ppm, by weight.The mixture is sulfated in a falling film reactor using the following conditions: mole ratios: conventional, i.e., slight excess of SO3 over fatty alcohol; typical SO3 concentrations: 4-5%, v/v in air, though no-air processes can also be used; typical fatty alcohol feed temperature: 10-25 deg. C (compare 55-60 which would be essential for tallow); cooling jacket water: 20-30 deg. C (compare 45 and above which would be needed for tallow alcohol, stearyl alcohol and the like); neutralization: 25-35 deg. C (compare 40-45 deg C for tallow alcohol); acid feed to neutralizer: 35-45 deg. C (compare tallow alcohol at 55 deg C). Final paste concentrations above 30% by weight are possible (compare tallow which typically sets up solid almost independently of paste concentration when there is no LAB or ethanol used).

The following two analytical methods for characterizing branching in the present invention surfactant compositions are useful:
1) Separation and Identification of Components in Fatty Alcohols prior to alkoxylation. The position and length of branching found in the precursor fatty alcohol materials is determined by GC/MS techniques [see: D. J. Harvey, Biomed, Environ. Mass Spectrom (1989). 18(9), 719-23; D. J. Harvey, J. M. Tiffany, J. Chromatogr. (1984), 301(1), 173-87; K. A. Karlsson, B. E. Samuelsson, G. O. Steen, Chem. Phys. Lipids (1973), 11(1), 17-38].
2) Identification of Separated Fatty Alcohol Components prior to alkoxylation by MS/MS techniques. The position and length of branching is also determinable by Ion Spray-MS/MS or FAB-MS/MS techniques on previously isolated fatty alcohol components.

Having identified isolated alcohol components, alkoxylation of the isolated alcohols yields standards useful for calibrating and interpreting GC chromatograms of mid-chain branched primary alkyl polyoxyalkylene surfactants.

The average total carbon atoms of the branched primary alkyl polyoxyalkylenes herein can be calculated from the hydroxyl value of the precursor fatty alcohol mix according to common procedures, such as outlined in "Bailey's Industrial Oil and Fat Products", Volume 2, Fourth Edition, edited by Daniel Swern, pp. 440-441.

## Claims

1. A method for manufacturing longer chain alkyl sulfate surfactant mixture compositions, said method comprising the steps of:
(a) sulfating with SO₃, preferably in a falling film reactor, a long chain aliphatic alcohol mixture having an average carbon chain length of at least 14.5 to 17.5, said alcohol mixture comprising at least 10 %, preferably at least 25%, more preferably at least 50%, still more preferably at least 75%, and most preferably at least 95%, of a mid-chain branched aliphatic alcohol having the formula: 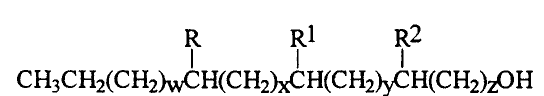 wherein the total number of carbon atoms in the branched primary alkyl moiety of this formula, including the R, R¹, and R² branching, is from 14 to 20, and wherein further for this alcohol mixture the average total number of carbon atoms in the branched primary alkyl moieties having the above formula is within the range of greater than 14.5 to 17.5, preferably greater than 15 to 17; R, R¹, and R² are each independently selected from hydrogen and C₁-C₃ alkyl, preferably methyl, provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; and w + x + y + z is from 8 to 14; and
(b) neutralizing the alkyl sulfate acid produced by step (a), preferably utilizing a base selected from the group consisting of potassium hydroxide, sodium hydroxide, ammonia monoethanolamine, triethanolamine, and mixtures thereof.

2. The method according to Claim 1 wherein the alcohol feed temperature for a step
(a) is less than 50°C, preferably within the range of from 10°C to 50°C, more preferably within the range of from 15°C to 45°C, and most preferably from 20°C to 35°C.

3. The process according to either of Claims 1 or 2 wherein the aliphatic alcohol sulfated in step (a) comprises at least 10%, preferably at least 25%, more preferably at least 50%, still more preferably at least 75%, and most preferably at least 95%, by weight of a mixture of two or more mid-chain branched alcohols having the formula: 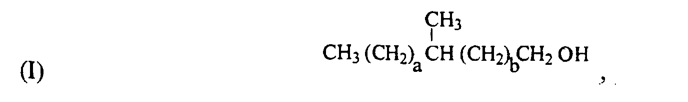 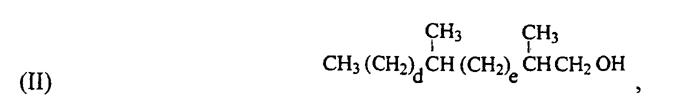 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;
wherein for this alcohol mixture the average total number of carbon atoms in the branched primary alkyl moieties having the above formulas is within the range of greater than 14.5 to 17.5.

4. A method for manufacturing longer chain alkyl alkoxylated sulfate surfactant mixture compositions, said method comprising the steps of:
(a) alkoxylating a long chain aliphatic alcohol mixture having an average carbon chain length of at least 14.5 to 17.5, said alcohol mixture comprising at least 10%, preferably at least 25%, more preferably at least 50%, still more preferably at least 75%, and most preferably at least 95%, of a mid-chain branched aliphatic alcohol having the formula: 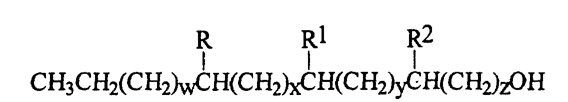 wherein the total number of carbon atoms in the branched primary alkyl moiety of this formula, including the R, R¹, and R² branching, is from 14 to 20, and wherein further for this alcohol mixture the average total number of carbon atoms in the branched primary alkyl moieties having the above formula is within the range of greater than 14.5 to 17.5, preferably greater than 15 to 17; R, R¹, and R² are each independently selected from hydrogen and C₁-C₃ alkyl, preferably methyl, provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; and w + x + y + z is from 8 to 14;
(b) sulfating the polyoxyalkylene alcohol produced by step (a) with SO₃; and
(c) neutralizing the alkyl alkoxylate sulfate acid produced by step (b), preferably utilizing a base selected from the group consisting of potassium hydroxide, sodium hydroxide, ammonia monoethanolamine, triethanolamine, and mixtures thereof.

5. The method according to Claim 4 wherein the polyoxyalkylene alcohol feed temperature for a step (b) is less than 50°C, preferably within the range of from 10°C to 50°C, more preferably within the range of from 15°C to 45°C, and most preferably from 20°C to 35°C.

6. The process according to either of Claims 4 or 5 wherein the aliphatic alcohol alkoxylated in step (a) comprises at least 10%, preferably at least 25%, more preferably at least 50%, still more preferably at least 75%, and most preferably at least 95%, by weight of a mixture of two or more mid-chain branched alcohols having the formula: 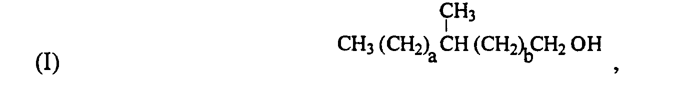 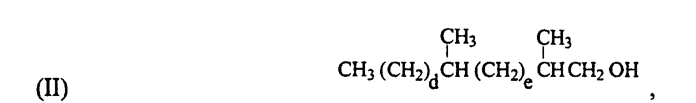 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from I to 10;
when d + e = 13, d is an integer from 2 to 12 and e is an integer from 1 to 11;
when d + e = 14, d is an integer from 2 to 13 and e is an integer from 1 to 12;
wherein for this alcohol mixture the average total number of carbon atoms in the branched primary alkyl moieties having the above formulas is within the range of greater than 14.5 to 17.5.

7. A method for manufacturing longer chain alkyl alkoxylated sulfate surfactant mixture compositions, said method comprising the steps of:
(a) sulfating with SO₃ a long chain aliphatic polyoxyalkylene alcohol mixture having an average aliphatic alcohol carbon chain length of at least 14.5 to 17.5, said polyoxyalkylene alcohol mixture comprising at least 10% (preferably at least 25%, more preferably at least 50%, still more preferably at least 75%, and most preferably at least 95%) of a mid-chain branched aliphatic polyoxyalkylene alcohol having the formula: 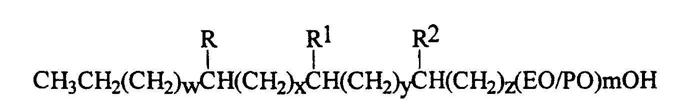 wherein the total number of carbon atoms in the branched primary alkyl moiety of this formula, including the R, R¹, and R³ branching, but not including the carbon atoms in the EO/PO alkoxy moiety, is from 14 to 20, and wherein further for this polyoxyalkylene alcohol mixture the average total number of carbon atoms in the branched primary alkyl moieties having the above formula is within the range of greater than 14.5 to 17.5, preferably from 15 to 17; R, R¹, and R² are each independently selected from hydrogen and C₁-C₃ alkyl, preferably methyl, provided R, R¹, and R² are not all hydrogen and, when z is 1, at least R or R¹ is not hydrogen; w is an integer from 0 to 13; x is an integer from 0 to 13; y is an integer from 0 to 13; z is an integer of at least 1; w + x + y + z is from 8 to 14; and EO/PO are alkoxy moieties, preferably selected from ethoxy, propoxy, and mixed ethoxy/propoxy groups, wherein m is at least 0.01, preferably within the range of from 0.1 to 30, more preferably from 0.5 to 10, and most preferably from 1 to 5; and
(b) neutralizing the alkyl alkoxylated sulfate acid produced by step (a), preferably utilizing a base selected from the group consisting of potassium hydroxide, sodium hydroxide, ammonia monoethanolamine, triethanolamine, and mixtures thereof.

8. The method according to Claim 7 wherein the polyoxyalkylene alcohol feed temperature for a step (a) is less than 50°C, preferably within the range of from 10°C to 50°C, more preferably within the range of from 15°C to 45°C, and most preferably from 20°C to 35°C.

9. The process acording to any of Claims 1-8 wherein the neutralization temperature is within the range of from 25°C to 35°C and the acid feed temperature into the neutralizer is within the range of from 35°C to 45°C.

10. The process acording to any of Claims 1-9 wherein the cooling jacket water temperature for the sulfation reactor used for step (a) is in the range of from 20°C to 30°C.

11. The process according to any of Claims 7-10 wherein the aliphatic polyoxyalkylene alcohol sulfated in step (a) comprises at least 10%, preferably at least 25%, more preferably at least 50%, still more preferably at least 75%, and most preferably at least 95%, by weight of a mixture of two or more mid-chain branched alcohols having the formula: 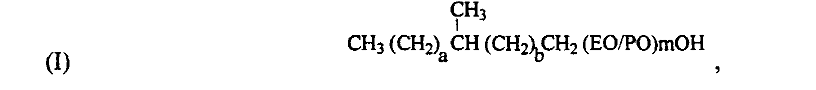 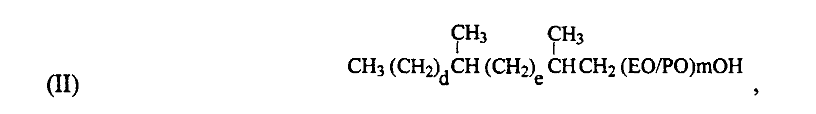 or mixtures thereof; wherein a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;
wherein for this polyoxyalkylene alcohol mixture the average total number of carbon atoms in the branched primary alkyl moieties having the above formulas is within the range of greater than 14.5 to 17.5; and
wherein EO/PO are alkoxy moieties, preferably selected from ethoxy, propoxy, and mixed ethoxy/propoxy groups, wherein m is at least 0.01, preferably within the range of from 0.1 to 30, more preferably from 0.5 to 10, and most preferably from 1 to 5.

## Patentansprüche

1. Verfahren zur Herstellung von längerkettigen Alkylsulfattensidmischungszusammensetzungen, wobei das Verfahren die Schritte umfaßt:
(a) Sulfatieren mit SO₃, bevorzugt in einem Fallfilmreaktor, einer langkettigen, aliphatischen Alkoholmischung mit einer mittleren Kohlenstoffkettenlänge von mindestens 14,5 bis 17,5, wobei die Alkoholmischung mindestens 10%, bevorzugt mindestens 25%, besonders bevorzugt mindestens 50%, ganz besonders bevorzugt mindestens 75%, und am meisten bevorzugt mindestens 95%, eines mittelkettig verzweigten aliphatischen Alkohols mit der Formel umfaßt: 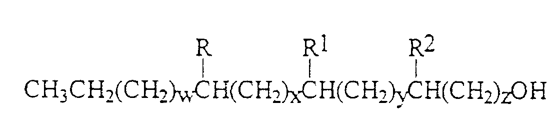 worin die Gesamtzahl der Kohlenstoffatome in der verzweigten primären Alkyleinheit dieser Formel, einschließlich der R-, R¹- und R²-Verzweigung, von 14 bis 20 reicht, und worin weiterhin für diese Alkoholmischung die mittlere Gesamtzahl der Kohlenstoffatome in den verzweigten primären Alkyleinheiten mit der obigen Formel innerhalb des Bereichs von größer als 14,5 bis 17,5, bevorzugt von größer als 15 bis 17, liegt; R, R¹ und R² jeweils unabhängig voneinander ausgewählt werden aus Wasserstoff und C₁-C₃-Alkyl, bevorzugt Methyl, vorausgesetzt, daß R, R¹ und R² nicht alle Wasserstoff sind und, wenn z 1 ist, mindestens R oder R¹ nicht Wasserstoff ist; w eine ganze Zahl von 0 bis 13 darstellt; x eine ganze Zahl von 0 bis 13 ist; y eine ganze Zahl von 0 bis 13 ist; z eine ganze Zahl von mindestens 1 darstellt; und w + x + y + z von 8 bis 14 reicht; und
(b) Neutralisieren der Alkylsulfatsäure, hergestellt durch Schritt (a), bevorzugt unter Verwendung einer Base, ausgewählt aus der Gruppe bestehend aus Kaliumhydroxid, Natriumhydroxid, Ammoniak, Monoethanolamin, Triethanolamin, und Mischungen davon.

2. Verfahren nach Anspruch 1, worin die Alkoholzugabetemperatur bei Schritt (a) niedriger als 50°C ist, bevorzugt innerhalb des Bereichs von 10°C bis 50°C, besonders bevorzugt innerhalb des Bereichs von 15°C bis 45°C, liegt, und am meisten bevorzugt von 20°C bis 35°C reicht.

3. Verfahren nach Anspruch 1 oder 2. worin der in Schritt (a) sulfatierte aliphatische Alkohol mindestens 10 Gew.-%, bevorzugt mindestens 25 Gew.-%, besonders bevorzugt mindestens 50 Gew.-%, ganz besonders bevorzugt mindestens 75 Gew.-%, und am meisten bevorzugt mindestens 95 Gew.-%, einer Mischung von zwei oder mehreren mittelkettig verzweigten Alkoholen mit der Formel umfaßt: 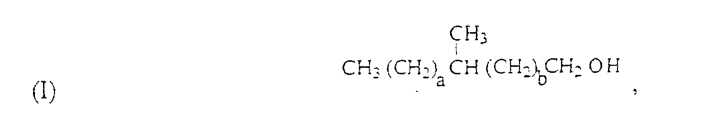 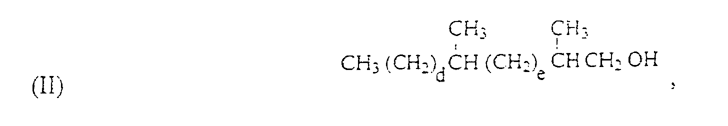 oder Mischungen davon; worin a, b, d, und e ganze Zahlen sind, a+b von 10 bis 16 reicht, d+e von 8 bis 14 reicht, und worin des weiteren gilt,
wenn a + b = 10, ist a eine ganze Zahl von 2 bis 9 und b eine ganze Zahl von 1 bis 8;
wenn a + b = 11, ist a eine ganze Zahl von 2 bis 10 und b eine ganze Zahl von 1 bis 9;
wenn a + b = 12, ist a eine ganze Zahl von 2 bis 11 und b eine ganze Zahl von 1 bis 10;
wenn a + b = 13, ist a eine ganze Zahl von 2 bis 12 und b eine ganze Zahl von 1 bis 11;
wenn a + b = 14, ist a eine ganze Zahl von 2 bis 13 und b eine ganze Zahl von 1 bis 12;
wenn a + b = 15, ist a eine ganze Zahl von 2 bis 14 und b eine ganze Zahl von 1 bis 13;
wenn a + b = 16, ist a eine ganze Zahl von 2 bis 15 und b eine ganze Zahl von 1 bis 14;
wenn d + e = 8, ist d eine ganze Zahl von 2 bis 7 und e eine ganze Zahl von 1 bis 6;
wenn d + e = 9, ist d eine ganze Zahl von 2 bis 8 und e eine ganze Zahl von 1 bis 7;
wenn d + e = 10, ist d eine ganze Zahl von 2 bis 9 und e eine ganze Zahl von 1 bis 8;
wenn d + e = 11, ist d eine ganze Zahl von 2 bis 10 und e eine ganze Zahl von 1 bis 9;
wenn d + e = 12, ist d eine ganze Zahl von 2 bis 11 und e eine ganze Zahl von 1 bis 10;
wenn d + e = 13, ist d eine ganze Zahl von 2 bis 12 und e eine ganze Zahl von 1 bis 11;
wenn d + e = 14, ist d eine ganze Zahl von 2 bis 13 und e eine ganze Zahl von 1 bis 12;
worin für diese Alkoholmischung die mittlere Gesamtzahl der Kohlenstoffatome in den verzweigten primären Alkyleinheiten mit den obigen Formeln innerhalb des Bereichs von größer als 14,5 bis 17,5 liegt.

4. Verfahren zur Herstellung längerkettiger alkoxylierter Alkylsulfattensidmischungszusammensetzungen, worin das Verfahren die Schritte umfaßt:
(a) Alkoxylieren einer langkettigen aliphatischen Alkoholmischung mit einer mittleren Kohlenstoffkettenlänge von mindestens 14,5 bis 17,5, wobei die Alkoholmischung mindestens 10%, bevorzugt mindestens 25%, besonders bevorzugt mindestens 50%, ganz besonders bevorzugt mindestens 75%, am meisten bevorzugt mindestens 95%, eines mittelkettig verzweigten aliphalischen Alkohols mit der Formel umfaßt: 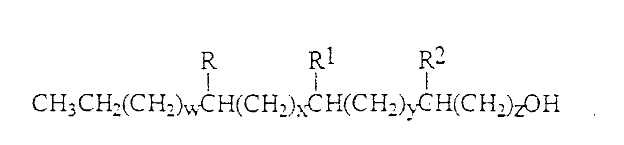 worin die Gesamtzahl der Kohlenstoffatome in der verzweigten primären Alkyleinheit dieser Formel, einschließlich der R-, R¹- und R²-Verzweigung, von 14 bis 20 reicht, und worin weiterhin für diese Alkoholmischung die mittlere Gesamtzahl der Kohlenstoffatome in den verzweigten primären Alkyleinheiten mit der obigen Formel innerhalb des Bereichs von größer als 14, 5 bis 17,5, bevorzugt von größer als 15 bis 17, liegt; R, R¹ und R² jeweils unabhängig voneinander ausgewählt werden aus Wasserstoff und C₁-C₃-Alkyl, bevorzugt Methyl, vorausgesetzt, daß R, R¹ und R² nicht alle Wasserstoff sind und, wenn z 1 ist, mindestens R oder R¹ nicht Wasserstoff ist; w eine ganze Zahl von 0 bis 13 darstellt; x eine ganze Zahl von 0 bis 13 ist; y eine ganze Zahl von 0 bis 13 ist; z eine ganze Zahl von mindestens 1 darstellt; und w + x + y + z von 8 bis 14 reicht;
(b) Sulfatieren des durch Schritt (a) hergestellten Polyoxyalkylenalkohols mit SO₃; und
(c) Neutralisieren der durch Schritt (b) hergestellten Alkylalkoxylatsulfatsäure, bevorzugt unter Verwendung einer Base, ausgewählt aus der Gruppe bestehend aus Kaliumhydroxid, Natriumhydroxid, Ammoniak, Monoethanolamin, Triethanolamin, und Mischungen davon.

5. Verfahen nach Anspruch 4, worin die Polyoxyalkylenzugabetemperatur bei Schritt (b) niedriger als 50°C ist, bevorzugt innerhalb des Bereichs von 10°C bis 50°C, besonders bevorzugt innerhalb des Bereichs von 15°C bis 45°C, liegt, und am meisten bevorzugt von 20°C bis 35°C reicht.

6. Verfahren nach Anspruch 4 oder 5, worin der in Schritt (a) alkoxylierte aliphatische Alkohol mindestens 10 Gew.-%, bevorzugt mindestens 25 Gew.-%, besonders bevorzugt mindestens 50 Gew.-%, ganz besonders bevorzugt mindestens 75 Gew.-%, und am meisten bevorzugt mindestens 95 Gew.-%, einer Mischung von zwei oder mehreren mittelkettig verzweigten Alkoholen mit der Formel umfaßt: 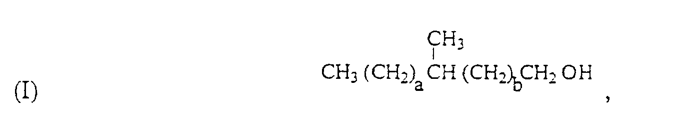 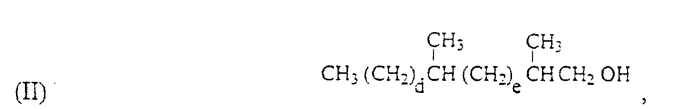 oder Mischungen davon; worin a, b, d und e ganze Zahlen sind, a + b von 10 bis 16 reicht, d + e von 8 bis 14 reicht und worin des weiteren gilt,
wenn a + b = 10, ist a eine ganze Zahl von 2 bis 9 und b eine ganze Zahl von 1 bis 8;
wenn a + b = 11, ist a eine ganze Zahl von 2 bis 10 und b eine ganze Zahl von 1 bis 9;
wenn a + b = 12, ist a eine ganze Zahl von 2 bis 11 und b eine ganze Zahl von 1 bis 10;
wenn a + b = 13, ist a eine ganze Zahl von 2 bis 12 und b eine ganze Zahl von 1 bis 11;
wenn a + b = 14, ist a eine ganze Zahl von 2 bis 13 und b eine ganze Zahl von 1 bis 12;
wenn a + b = 15 ist a eine ganze Zahl von 2 bis 14 und b eine ganze Zahl von 1 bis 13;
wenn a + b = 16, ist a eine ganze Zahl von 2 bis 15 und b eine ganze Zahl von 1 bis 14;
wenn d + e = 8, ist d eine ganze Zahl von 2 bis 7 und e eine ganze Zahl von 1 bis 6;
wenn d + e = 9, ist d eine ganze Zahl von 2 bis 8 und e eine ganze Zahl von 1 bis 7;
wenn d + e = 10, ist d eine ganze Zahl von 2 bis 9 und e eine ganze Zahl von 1 bis 8;
wenn d + e = 11, ist d eine ganze Zahl von 2 bis 10 und e eine ganze Zahl von 1 bis 9;
wenn d + e = 12, ist d eine ganze Zahl von 2 bis 11 und e eine ganze Zahl von 1 bis 10;
wenn d + e = 13, ist d eine ganze Zahl von 2 bis 12 und e eine ganze Zahl von 1 bis 11;
wenn d + e = 14, ist d eine ganze Zahl von 2 bis 13 und e eine ganze Zahl von 1 bis 12;
worin für diese Alkoholmischung die mittlere Gesamtzahl der Kohlenstoffatome in den verzweigten primären Alkyleinheiten mit den obigen Formeln innerhalb des Bereichs von größer als 14,5 bis 17,5 liegt.

7. Verfahren zur Herstellung längerkettiger alkoxylierter Alkylsulfattensidmischungszusammensetzungen, wobei das Verfahren die Schritte umfaßt:
(a) Sulfatieren mit SO₃ einer langkettigen aliphatischen Polyoxyalkylenalkoholmischung mit einer mittleren aliphatischen Alkoholkohlenstoffkettenlänge von mindestens 14,5 bis 17,5, wobei die Polyoxyalkylenalkoholmischung mindestens 10% (bevorzugt mindestens 25%, besonders bevorzugt mindestens 50%, ganz besonders bevorzugt mindestens 75%, und am meisten bevorzugt mindestens 95%) eines mittelkettig verzweigten aliphatischen Polyoxyalkylenalkohols mit der Formel umfaßt: 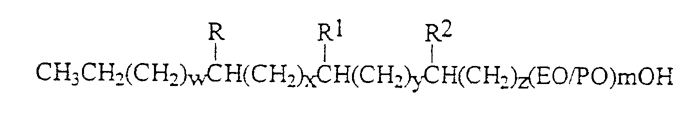 worin die Gesamtzahl der Kohlenstoffatome in der verzweigten primären Alkyleinheit dieser Formel, einschließlich der R-, R¹- und R²-Verzweigung, allerdings ohne die Kohlenstoffatome in der EO/PO-Alkoxyeinheit einzuschließen, von 14 bis 20 reicht, und worin weiterhin für diese Polyoxyalkylenalkoholmischung die mittlere Gesamtzahl der Kohlenstoffatome in den verzweigten primären Alkyleinheiten mit der obigen Formel innerhalb des Bereichs von größer als 14,5 bis 17,5, bevorzugt von 15 bis 17, liegt; R, R¹ und R² jeweils unabhängig voneinander ausgewählt werden aus Wasserstoff und C₁-C₃-Alkyl, bevorzugt Methyl, vorausgesetzt das R, R¹ und R² nicht alle Wasserstoff sind und, wenn z 1 ist, mindestens R oder R¹ nicht Wasserstoff ist; w eine ganze Zahl von 0 bis 13 darstellt; x eine ganze Zahl von 0 bis 13 ist; y eine ganze Zahl von 0 bis 13 ist; z eine ganze Zahl von mindestens 1 darstellt; w + x + y + z von 8 bis 14 reicht; und EO/PO Alkoxyeinheiten darstellen, bevorzugt ausgewählt aus Ethoxy-, Propoxy-, und gemischten Ethoxy-/Propoxygruppen, worin m mindestens 0,01 ist, bevorzugt innerhalb des Bereichs von 0,1 bis 30 liegt, besonders bevorzugt von 0,5 bis 10, und am meisten bevorzugt von 1 bis 5, reicht; und
(b) Neutralisieren der durch Schritt (a) hergestellten alkoxyierten Alkylsulfatsäure, bevorzugt unter Verwendung einer Base, ausgewählt aus der Gruppe bestehend aus Kaliumhydroxid, Natriumhydroxid, Ammoniak, Monoethanolamin, Triethanolamin, und Mischungen davon.

8. Verfahren nach Anspruch 7, worin die Polyoxyalkylenalkoholzugabetemperatur bei Schritt (a) niedriger als 50°C ist, bevorzugt innerhalb des Bereichs von 10°C bis 50°C, besonders bevorzugt innerhalb des Bereichs von 15°C bis 45°C, liegt, und am meisten bevorzugt von 20°C bis 35°C reicht.

9. Verfahren nach mindestens einem der Ansprüche 1 bis 8, worin die Neutralisierungtemperatur innerhalb des Bereichs von 25°C bis 35°C liegt und die Temperatur der Säurezugabe in den Neutralisator innerhalb des Bereichs von 35°C bis 45°C liegt.

10. Verfahren nach mindestens einem der Ansprüche 1 bis 9, worin die Kühlmantelwassertemperatur des in dem Schritt (a) verwendeten Sulfatierungsreaktors in dem Bereich von 20°C bis 30°C liegt.

11. Verfahren nach mindestens einem der Ansprüche 7 bis 10, worin der in Schritt (a) sulfatierte aliphatische Polyoxyalkylenalkohol mindestens 10 Gew.-%, bevorzugt mindestens 25 Gew.-%, besonders bevorzugt mindestens 50 Gew.-%, ganz besonders bevorzugt mindestens 75 Gew.-%, und am meisten bevorzugt mindestens 95 Gew.-%, einer Mischung von zwei oder mehreren mittelkettig verzweigten Alkoholen mit der Formel umfaßt: 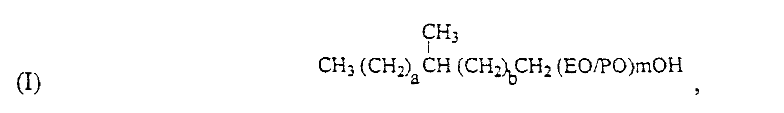 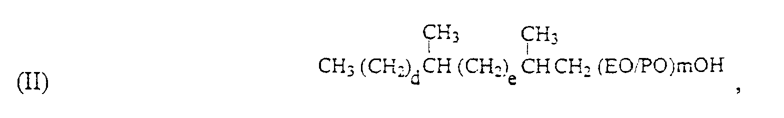 oder Mischungen davon; worin a, b, d und e ganze Zahlen sind, a + b von 10 bis 16 reicht, d + e von 8 bis 14 reicht, und worin des weiteren gilt,
wenn a + b = 10, ist a eine ganze Zahl von 2 bis 9 und b eine ganze Zahl von 1 bis 8;
wenn a + b = 11, ist a eine ganze Zahl von 2 bis 10 und b eine ganze Zahl von 1 bis 9;
wenn a + b = 12, ist a eine ganze Zahl von 2 bis 11 und b eine ganze Zahl von 1 bis 10;
wenn a + b = 13, ist a eine ganze Zahl von 2 bis 12 und b eine ganze Zahl von 1 bis 11;
wenn a + b = 14, ist a eine ganze Zahl von 2 bis 13 und b eine ganze Zahl von 1 bis 12;
wenn a + b = 15, ist a eine ganze Zahl von 2 bis 14 und b eine ganze Zahl von 1 bis 13;
wenn a + b = 16, ist a eine ganze Zahl von 2 bis 15 und b eine ganze Zahl von 1 bis 14;
wenn d + e = 8, ist d eine ganze Zahl von 2 bis 7 und e eine ganze Zahl von 1 bis 6;
wenn d + e = 9, ist d eine ganze Zahl von 2 bis 8 und e eine ganze Zahl von 1 bis 7;
wenn d + e = 10, ist d eine ganze Zahl von 2 bis 9 und e eine ganze Zahl von 1 bis 8;
wenn d + e = 11, ist d eine ganze Zahl von 2 bis 10 und e eine ganze Zahl von 1 bis 9;
wenn d + e = 12, ist d eine ganze Zahl von 2 bis 11 und e eine ganze Zahl von 1 bis 10;
wenn d + e = 13, ist d eine ganze Zahl von 2 bis 12 und e eine ganze Zahl von 1 bis 11;
wenn d + e = 14, ist d eine ganze Zahl von 2 bis 13 und e eine ganze Zahl von 1 bis 12;
worin für diese Polyoxyalkylenalkoholmischung die mittlere Gesamtzahl der Kohlenstoffatome in den verzweigten primären Alkyleinheiten mit den obigen Formeln innerhalb des Bereichs von größer als 14,5 bis 17,5 liegt; und
worin EP/PO Alkoxyeinheiten darstellen, bevorzugt ausgewählt aus Ethoxy-, Propoxy-, und gemischten Ethoxy-/Propoxygruppen, worin m mindestens 0,01 ist, bevorzugt innerhalb des Bereichs von 0,1 bis 30 liegt, besonders bevorzugt von 0,5 bis 10, und am meisten bevorzugt von 1 bis 5, reicht.

## Revendications

1. Procédé pour fabriquer des compositions de mélange de tensioactifs alkylsulfates à chaîne plus longue, ledit procédé comprenant les étapes consistant à :
(a) sulfater avec du SO₃, de préférence dans un réacteur à film tombant, un mélange d'alcools aliphatiques à longue chaîne ayant une longueur moyenne de chaîne carbonée d'au moins 14,5 à 17,5, ledit mélange d'alcools comprenant au moins 10 %, de préférence au moins 25 %, mieux encore au moins 50 %, plus particulièrement au moins 75 % et tout spécialement au moins 95 % d'un alcool aliphatique ramifié à chaîne moyenne de formule : 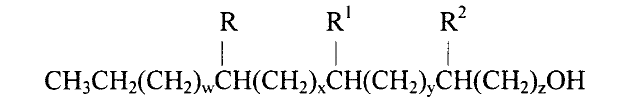 dans laquelle le nombre total d'atomes de carbone dans le fragment alkyle primaire ramifié de cette formule, y compris les ramifications R, R¹ et R², est de 14 à 20, et où en outre, pour ce mélange d'alcools, le nombre total moyen d'atomes de carbone dans les fragments alkyle primaires ramifiés de formule ci-dessus est situé dans la plage allant de plus de 14,5 à 17,5, de préférence de plus de 15 à 17 ; chacun de R, R¹, R² est indépendamment choisi parmi l'hydrogène et les radicaux alkyle en C₁ à C₃, de préférence méthyle, pourvu que R, R¹ et R² ne soient pas tous l'hydrogène et, quand z vaut 1, qu'au moins l'un de R et R¹ ne soit pas l'hydrogène ; w est un entier de 0 à 13 ; x est un entier de 0 à 13 ; y est un entier de 0 à 13 ; z est un entier valant au moins 1 ; et w + x + y + z vaut de 8 à 14 ; et
(b) neutraliser l'alkylsulfate acide produit par l'étape (a), de préférence en utilisant une base choisie dans l'ensemble constitué par l'hydroxyde de potassium, l'hydroxyde de sodium, l'ammoniac, la monoéthanolamine, la triéthanolamine, et leurs mélanges.

2. Procédé selon la revendication 1, dans lequel la température d'introduction de l'alcool pour l'étape (a) est inférieure à 50°C, de préférence située dans la plage allant de 10°C à 50°C, mieux encore dans la plage allant de 15°C à 45°C, et tout spécialement de 20°C à 35°C.

3. Procédé selon l'une ou l'autre des revendications 1 et 2, dans lequel l'alcool aliphatique sulfaté dans l'étape (a) comprend au moins 10 %, de préférence au moins 25 %, mieux encore au moins 50 %, plus particulièrement au moins 75 %, et tout spécialement au moins 95 % en poids d'un mélange de deux ou plus de deux alcools ramifiés à chaîne moyenne de formule : 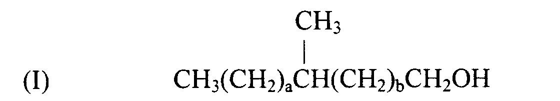 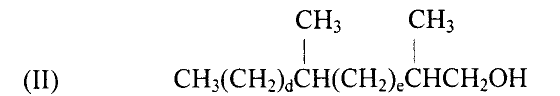 ou de leurs mélanges ; où a, b, d et e sont des entiers, a+b vaut de 10 à 16, d+e vaut de 8 à 14, et où, en outre :
quand a + b = 10, a est un entier de 2 à 9 et b est un entier de 1 à 8 ;
quand a + b = 11, a est un entier de 2 à 10 et b est un entier de 1 à 9 ;
quand a + b = 12, a est un entier de 2 à 11 et b est un entier de 1 à 10 ;
quand a + b = 13, a est un entier de 2 à 12 et b est un entier de 1 à 11 ;
quand a + b = 14, a est un entier de 2 à 13 et b est un entier de 1 à 12 ;
quand a + b = 15, a est un entier de 2 à 14 et b est un entier de 1 à 13 ;
quand a + b = 16, a est un entier de 2 à 15 et b est un entier de 1 à 14 ;
quand d + e = 8, d est un entier de 2 à 7 et e est un entier de 1 à 6 ;
quand d + e = 9, d est un entier de 2 à 8 et e est un entier de 1 à 7 ;
quand d + e = 10, d est un entier de 2 à 9 et e est un entier de 1 à 8 ;
quand d + e = 11, d est un entier de 2 à 10 et e est un entier de 1 à 9 ;
quand d + e = 12, d est un entier de 2 à 11 et e est un entier de 1 à 10 ;
quand d + e = 13, d est un entier de 2 à 12 et e est un entier de 1 à 11 ;
quand d + e = 14, d est un entier de 2 à 13 et e est un entier de 1 à 12 ;
où, pour ce mélange d'alcools, le nombre total moyen d'atomes de carbone dans les fragments alkyle primaires ramifiés ayant les formules ci-dessus est situé dans la plage allant de plus de 14,5 à 17,5.

4. Procédé pour fabriquer des compositions de mélange de tensioactifs alkylsulfates alcoxylés à chaîne plus longue, ledit procédé comprenant les étapes consistant à :
(a) alcoxyler un mélange d'alcools aliphatiques à longue chaîne ayant une longueur de chaîne carbonée moyenne d'au moins 14,5 à 17,5, ledit mélange d'alcools comprenant au moins 10 %, de préférence au moins 25 %, mieux encore au moins 50 %, plus particulièrement au moins 75 % et tout spécialement au moins 95 % d'un alcool aliphatique ramifié à chaîne moyenne de formule : 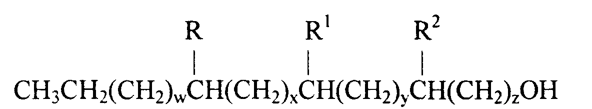 dans laquelle le nombre total d'atomes de carbone dans le fragment alkyle primaire ramifié de cette formule, y compris les ramifications R, R¹ et R², est de 14 à 20, et où en outre, pour ce mélange d'alcools, le nombre total moyen d'atomes de carbone dans les fragments alkyle primaires ramifiés ayant la formule ci-dessus est situé dans la plage allant de plus de 14,5 à 17,5, de préférence de plus de 15 à 17 ; chacun de R, R¹, R² est indépendamment choisi parmi l'hydrogène et les radicaux alkyle en C₁ à C₃, de préférence méthyle, pourvu que R, R¹ et R² ne soient pas tous l'hydrogène et, quand z vaut 1, qu'au moins l'un de R et R¹ ne soit pas l'hydrogène ; w est un entier de 0 à 13 ; x est un entier de 0 à 13 ; y est un entier de 0 à 13 ; z est un entier valant au moins 1 ; et w + x + y + z vaut de 8 à 14;
(b) sulfater le polyoxyalkylène-alcool produit par l'étape (a) avec du SO₃ ; et
(c) neutraliser l'alkylsulfate acide alcoxylé produit par l'étape (b), de préférence en utilisant une base choisie dans l'ensemble constitué par l'hydroxyde de potassium, l'hydroxyde de sodium, l'ammoniac, la monoéthanolamine, la triéthanolamine, et leurs mélanges.

5. Procédé selon la revendication 4, dans lequel la température d'introduction du polyoxyalkylène-alcool pour l'étape (b) est inférieure à 50°C, de préférence située dans la plage allant de 10°C à 50°C, mieux encore dans la plage allant de 15°C à 45°C, et tout spécialement de 20°C à 35°C.

6. Procédé selon l'une ou l'autre des revendications 4 et 5, dans lequel l'alcool aliphatique alcoxylé dans l'étape (a) comprend au moins 10 %, de préférence au moins 25 %, mieux encore au moins 50 %, plus particulièrement au moins 75 %, et tout spécialement au moins 95 % en poids d'un mélange de deux ou plus de deux alcools ramifiés à chaîne moyenne de formule : 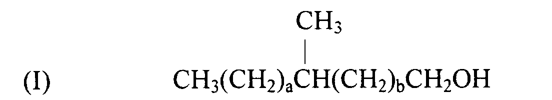 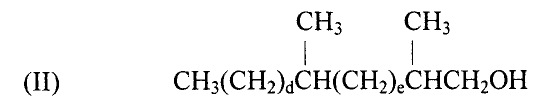 ou de leurs mélanges ; où a, b, d et e sont des entiers, a+b vaut de 10 à 16, d+e vaut de 8 à 14, et où, en outre :
quand a + b = 10, a est un entier de 2 à 9 et b est un entier de 1 à 8 ;
quand a + b = 11, a est un entier de 2 à 10 et b est un entier de 1 à 9 ;
quand a + b = 12, a est un entier de 2 à 11 et b est un entier de 1 à 10 ;
quand a + b = 13, a est un entier de 2 à 12 et b est un entier de 1 à 11 ;
quand a + b = 14, a est un entier de 2 à 13 et b est un entier de 1 à 12 ;
quand a + b = 15, a est un entier de 2 à 14 et b est un entier de 1 à 13 ;
quand a + b = 16, a est un entier de 2 à 15 et b est un entier de 1 à 14 ;
quand d + e = 8, d est un entier de 2 à 7 et e est un entier de 1 à 6 ;
quand d + e = 9, d est un entier de 2 à 8 et e est un entier de 1 à 7 ;
quand d + e = 10, d est un entier de 2 à 9 et e est un entier de 1 à 8 ;
quand d + e = 11, d est un entier de 2 à 10 et e est un entier de 1 à 9 ;
quand d + e = 12, d est un entier de 2 à 11 et e est un entier de 1 à 10 ;
quand d + e = 13, d est un entier de 2 à 12 et e est un entier de 1 à 11 ;
quand d + e = 14, d est un entier de 2 à 13 et e est un entier de 1 à 12 ;
où, pour ce mélange d'alcools, le nombre total moyen d'atomes de carbone dans les fragments alkyle primaires ramifiés ayant les formules ci-dessus est situé dans la plage allant de plus de 14,5 à 17,5.

7. Procédé pour fabriquer des compositions de mélange de tensioactifs alkylsulfates alcoxylés à chaîne plus longue, ledit procédé comprenant les étapes consistant à :
(a) sulfater avec du SO₃ un mélange de polyoxyalkylène-alcools aliphatiques à longue chaîne ayant une longueur moyenne de chaîne carbonée d'alcool aliphatique d'au moins 14,5 à 17,5, ledit mélange de polyoxyalkylène-alcools comprenant au moins 10 % (de préférence au moins 25 %, mieux encore au moins 50 %, plus particulièrement au moins 75 % et tout spécialement au moins 95 %) d'un polyoxyalkylène-alcool aliphatique ramifié à chaîne moyenne de formule : 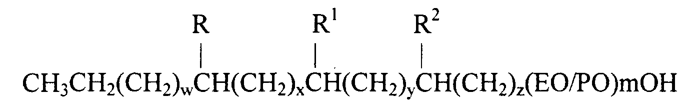 dans laquelle le nombre total d'atomes de carbone dans le fragment alkyle primaire ramifié de cette formule, y compris les ramifications R, R¹ et R², mais à l'exclusion des atomes de carbone du fragment alcoxy EO/PO, est de 14 à 20, et où en outre, pour ce mélange de polyoxyalkylène-alcools, le nombre total moyen d'atomes de carbone dans les fragments alkyle primaires ramifiés de formule ci-dessus est situé dans la plage allant de plus de 14,5 à 17,5, de préférence de 15 à 17 ; chacun de R, R¹, R² est indépendamment choisi parmi l'hydrogène et les radicaux alkyle en C₁ à C₃, de préférence méthyle, pourvu que R, R¹ et R² ne soient pas tous l'hydrogène et, quand z vaut 1, qu'au moins l'un de R et R¹ ne soit pas l'hydrogène ; w est un entier de 0 à 13 ; x est un entier de 0 à 13 ; y est un entier de 0 à 13 ; z est un entier valant au moins 1 ; w + x + y + z vaut de 8 à 14 ; et les fragments EO/PO sont des fragments alcoxy de préférence choisis parmi les groupes éthoxy, propoxy, et éthoxy/propoxy mixtes, où m vaut au moins 0,01 et est de préférence situé dans la plage allant de 0,1 à 30, mieux encore de 0,5 à 10, et tout spécialement de 1 à 5 ; et
(b) neutraliser l'alkylsulfate alcoxylé acide produit par l'étape (a), de préférence en utilisant une base choisie dans l'ensemble constitué par l'hydroxyde de potassium, l'hydroxyde de sodium, l'ammoniac, la monoéthanolamine, la triéthanolamine, et leurs mélanges.

8. Procédé selon la revendication 7, dans lequel la température d'introduction du polyoxyalkylène-alcool pour l'étape (a) est inférieure à 50°C, de préférence située dans la plage allant de 10°C à 50°C, mieux encore dans la plage allant de 15°C à 45°C, et tout spécialement de 20°C à 35°C.

9. Procédé selon l'une quelconque des revendications 1 à 8, dans lequel la température de neutralisation est située dans la plage allant de 25°C à 35°C et la température d'introduction de l'acide dans le dispositif de neutralisation est située dans la plage allant de 35°C à 45°C.

10. Procédé selon l'une quelconque des revendications 1 à 9, dans lequel la température de l'eau de la chemise de refroidissement pour le réacteur de sulfatation utilisé pour l'étape (a) est située dans la plage allant de 20°C à 30°C.

11. Procédé selon l'une quelconque des revendications 7 à 10, dans lequel le polyoxyalkylène-alcool aliphatique sulfaté dans l'étape (a) comprend au moins 10 %, de préférence au moins 25 %, mieux encore au moins 50 %, plus particulièrement au moins 75 %, et tout spécialement au moins 95 % en poids d'un mélange de deux ou plus de deux alcools ramifiés à chaîne moyenne de formule : 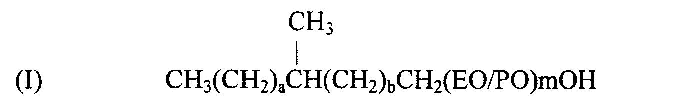 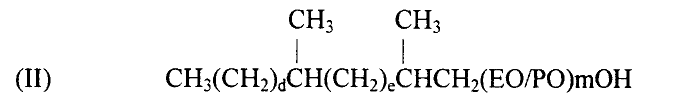 ou de leurs mélanges ; où a, b, d et e sont des entiers, a+b vaut de 10 à 16, d+e vaut de 8 à 14, et où, en outre :
quand a + b = 10, a est un entier de 2 à 9 et b est un entier de 1 à 8 ;
quand a + b = 11, a est un entier de 2 à 10 et b est un entier de 1 à 9 ;
quand a + b = 12, a est un entier de 2 à 11 et b est un entier de 1 à 10 ;
quand a + b = 13, a est un entier de 2 à 12 et b est un entier de 1 à 11 ;
quand a + b = 14, a est un entier de 2 à 13 et b est un entier de 1 à 12 ;
quand a + b = 15, a est un entier de 2 à 14 et b est un entier de 1 à 13 ;
quand a + b = 16, a est un entier de 2 à 15 et b est un entier de 1 à 14 ;
quand d + e = 8, d est un entier de 2 à 7 et e est un entier de 1 à 6 ;
quand d + e = 9, d est un entier de 2 à 8 et e est un entier de 1 à 7;
quand d + e = 10, d est un entier de 2 à 9 et e est un entier de 1 à 8;
quand d + e = 11, d est un entier de 2 à 10 et e est un entier de 1 à 9 ;
quand d + e = 12, d est un entier de 2 à 11 et e est un entier de 1 à 10;
quand d + e = 13, d est un entier de 2 à 12 et e est un entier de 1 à 11;
quand d + e = 14, d est un entier de 2 à 13 et e est un entier de 1 à 12 ;
où, pour ce mélange de polyoxyalkylène-alcools, le nombre total moyen d'atomes de carbone dans les fragments alkyle primaires ramifiés ayant les formules ci-dessus est situé dans la plage allant de plus de 14,5 à 17,5 ; et
où les fragments EO/PO sont des fragments alcoxy, de préférence choisis parmi les groupes éthoxy, propoxy et éthoxy/propoxy mixtes, où m vaut au moins 0,01, et de préférence est situé dans la plage allant de 0,1 à 30, mieux encore de 0,5 à 10, et tout spécialement de 1 à 5.
